# EUROPEAN PATENT APPLICATION

(11) **EP 4 074 825 A1**
(43) Date of publication of application: **19.10.2022**
(21) Application number: 20899155.4
(22) Date of filing: 08.12.2020
(51) Int. Cl.: C12N 15/113, A61K 35/76, A61K 38/50, A61K 48/00, C12N 1/15, C12N 1/19, C12N 1/21, C12N 5/10, C12N 15/09, C12N 15/63, C12P 19/34

(54) **ANTISENSE GUIDE RNA WITH ADDED FUNCTIONAL REGION FOR EDITING TARGET RNA**

(30) Priority: 09.12.2019 JP 2019222437
(71) Applicant: Astellas Pharma Inc., Chuo-ku Tokyo 103-8411 (JP)
(72) Inventor: YOSHIMI, Eiji, Tokyo 103-8411 (JP); MORIYA, Yukari, Tokyo 103-8411 (JP); MANDA, Mariko, Tokyo 103-8411 (JP); GOTOH, Takayasu, Tokyo 103-8411 (JP); ARAI, Takatomo, Tokyo 103-8411 (JP)
(74) Representative: Hoffmann Eitle
(86) International application number: PCT/JP2020/045707
(87) International publication number: WO 2021/117729

(57) **Abstract**

Problem: Providing antisense guide RNA for editing target RNA by ADAR. Solution: Antisense guide RNA, for editing target RNA by ADAR, wherein: at least one functional region and an antisense region that is complementary to part of the target RNA and forms a double strand with the target RNA are included; and the at least one functional region is joined to the antisense region and substantially does not include an ADAR-recruiting base sequence. Representative drawing: None

### SEQUENCE LISTING

SEQUENCE LISTING

<110>  Astellas Pharma Inc.

<120>  A target RNA-editing antisense-type guide RNA to which a
       functional sequence is added

<130>  A20028A00

<150>  JP2019-222437
<151>  2019-12-09

<160>  52

<170>  PatentIn version 3.5

<210>  1
<211>  27
<212>  RNA
<213>  Artificial Sequence

<220>
<223>  Synthetic RNA

<400>  1
gugcucgcuu cggcagcaca uauacua                                          27


<210>  2
<211>  20
<212>  RNA
<213>  Artificial Sequence

<220>
<223>  Synthetic RNA

<400>  2
gcggacuucg guccgcuuuu                                                  20


<210>  3
<211>  216
<212>  RNA
<213>  Artificial Sequence

<220>
<223>  Synthetic RNA

<400>  3
auacuuaccu ggcaggggag auaccaugau cacgaaggug guuuucccag ggcgaggcuu      60

auccauugca cuccggaugu gcugaccccu gcgauuuccc caaauguggg aaacucgacu     120

gcauaauuug ugguaguggg ggacugcguu cgcgcuuucc ccugacuuuc uggaguuuca     180

aaaguagacu guacgcuaag ggucauaucu uuuuuu                               216


<210>  4
<211>  149
<212>  RNA
<213>  Artificial Sequence

<220>
<223>  Synthetic RNA

<400>  4
aauuuuugga gcagguuuuc ugacuucggu cggaaaaccc cucccaauuu cacuggucua      60

caaugaaagc aaaacaguuc ucuuccccgc uccccggugu gugagagggg cuuugauccu     120

ucucugguuu ccuaggaaac gcguaugug                                       149


<210>  5
<211>  118
<212>  RNA
<213>  Artificial Sequence

<220>
<223>  Synthetic RNA

<400>  5
gucuacggcc auaccacccu gaacgcgccc gaucucgucu gaucucggaa gcuaagcagg      60

gucgggccug guuaguacuu ggaugggaga ccgccuggga auaccgggug cuguaggc       118


<210>  6
<211>  24
<212>  RNA
<213>  Artificial Sequence

<220>
<223>  Synthetic RNA

<400>  6
ggguuugggu uuggguuugg guuu                                             24


<210>  7
<211>  24
<212>  RNA
<213>  Artificial Sequence

<220>
<223>  Synthetic RNA

<400>  7
agagggcccu gaagagggcc cuuu                                             24


<210>  8
<211>  2127
<212>  DNA
<213>  Homo sapiens


<220>
<221>  CDS
<222>  (16)..(2118)

<400>  8
gaattcgccg ccacc atg gat ata gaa gat gaa gaa aac atg agt tcc agc      51
                 Met Asp Ile Glu Asp Glu Glu Asn Met Ser Ser Ser
                 1               5                   10

agc act gat gtg aag gaa aac cgc aat ctg gac aac gtg tcc ccc aag          99
Ser Thr Asp Val Lys Glu Asn Arg Asn Leu Asp Asn Val Ser Pro Lys
        15                  20                  25

gat ggc agc aca cct ggg cct ggc gag ggc tct cag ctc tcc aat ggg         147
Asp Gly Ser Thr Pro Gly Pro Gly Glu Gly Ser Gln Leu Ser Asn Gly
    30                  35                  40

ggt ggt ggt ggc ccc ggc aga aag cgg ccc ctg gag gag ggc agc aat         195
Gly Gly Gly Gly Pro Gly Arg Lys Arg Pro Leu Glu Glu Gly Ser Asn
45                  50                  55                  60

ggc cac tcc aag tac cgc ctg aag aaa agg agg aaa aca cca ggg ccc         243
Gly His Ser Lys Tyr Arg Leu Lys Lys Arg Arg Lys Thr Pro Gly Pro
                65                  70                  75

gtc ctc ccc aag aac gcc ctg atg cag ctg aat gag atc aag cct ggt         291
Val Leu Pro Lys Asn Ala Leu Met Gln Leu Asn Glu Ile Lys Pro Gly
            80                  85                  90

ttg cag tac aca ctc ctg tcc cag act ggg ccc gtg cac gcg cct ttg         339
Leu Gln Tyr Thr Leu Leu Ser Gln Thr Gly Pro Val His Ala Pro Leu
        95                  100                 105

ttt gtc atg tct gtg gag gtg aat ggc cag gtt ttt gag ggc tct ggt         387
Phe Val Met Ser Val Glu Val Asn Gly Gln Val Phe Glu Gly Ser Gly
    110                 115                 120

ccc aca aag aaa aag gca aaa ctc cat gct gct gag aag gcc ttg agg         435
Pro Thr Lys Lys Lys Ala Lys Leu His Ala Ala Glu Lys Ala Leu Arg
125                 130                 135                 140

tct ttc gtt cag ttt cct aat gcc tct gag gcc cac ctg gcc atg ggg         483
Ser Phe Val Gln Phe Pro Asn Ala Ser Glu Ala His Leu Ala Met Gly
                145                 150                 155

agg acc ctg tct gtc aac acg gac ttc aca tct gac cag gcc gac ttc         531
Arg Thr Leu Ser Val Asn Thr Asp Phe Thr Ser Asp Gln Ala Asp Phe
            160                 165                 170

cct gac acg ctc ttc aat ggt ttt gaa act cct gac aag gcg gag cct         579
Pro Asp Thr Leu Phe Asn Gly Phe Glu Thr Pro Asp Lys Ala Glu Pro
        175                 180                 185

ccc ttt tac gtg ggc tcc aat ggg gat gac tcc ttc agt tcc agc ggg         627
Pro Phe Tyr Val Gly Ser Asn Gly Asp Asp Ser Phe Ser Ser Ser Gly
    190                 195                 200

gac ctc agc ttg tct gct tcc ccg gtg cct gcc agc cta gcc cag cct         675
Asp Leu Ser Leu Ser Ala Ser Pro Val Pro Ala Ser Leu Ala Gln Pro
205                 210                 215                 220

cct ctc cct gtc tta cca cca ttc cca ccc ccg agt ggg aag aat ccc         723
Pro Leu Pro Val Leu Pro Pro Phe Pro Pro Pro Ser Gly Lys Asn Pro
                225                 230                 235

gtg atg atc ttg aac gaa ctg cgc cca gga ctc aag tat gac ttt ctc         771
Val Met Ile Leu Asn Glu Leu Arg Pro Gly Leu Lys Tyr Asp Phe Leu
            240                 245                 250

tcc gag agc ggg gag agc cat gcc aag agc ttc gtc atg tct gtg gtc         819
Ser Glu Ser Gly Glu Ser His Ala Lys Ser Phe Val Met Ser Val Val

255                 260                 265

gtg gat ggt cag ttc ttt gaa ggc tcg ggg aga aac aag aag ctt gcc      867
Val Asp Gly Gln Phe Phe Glu Gly Ser Gly Arg Asn Lys Lys Leu Ala
    270                 275                 280

aag gcc cgg gct gcg cag tct gcc ctg gcc gcc att ttt aac ttg cac      915
Lys Ala Arg Ala Ala Gln Ser Ala Leu Ala Ala Ile Phe Asn Leu His
285                 290                 295                 300

ttg gat cag acg cca tct cgc cag cct att ccc agt gag ggt ctt cag      963
Leu Asp Gln Thr Pro Ser Arg Gln Pro Ile Pro Ser Glu Gly Leu Gln
                305                 310                 315

ctg cat tta ccg cag gtt tta gct gac gct gtc tca cgc ctg gtc ctg     1011
Leu His Leu Pro Gln Val Leu Ala Asp Ala Val Ser Arg Leu Val Leu
            320                 325                 330

ggt aag ttt ggt gac ctg acc gac aac ttc tcc tcc cct cac gct cgc     1059
Gly Lys Phe Gly Asp Leu Thr Asp Asn Phe Ser Ser Pro His Ala Arg
        335                 340                 345

aga aaa gtg ctg gct gga gtc gtc atg aca aca ggc aca gat gtt aaa     1107
Arg Lys Val Leu Ala Gly Val Val Met Thr Thr Gly Thr Asp Val Lys
    350                 355                 360

gat gcc aag gtg ata agt gtt tct aca gga aca aaa tgt att aat ggt     1155
Asp Ala Lys Val Ile Ser Val Ser Thr Gly Thr Lys Cys Ile Asn Gly
365                 370                 375                 380

gaa tac atg agt gat cgt ggc ctt gca tta aat gac tgc cat gca gaa     1203
Glu Tyr Met Ser Asp Arg Gly Leu Ala Leu Asn Asp Cys His Ala Glu
                385                 390                 395

ata ata tct cgg aga tcc ttg ctc aga ttt ctt tat aca caa ctt gag     1251
Ile Ile Ser Arg Arg Ser Leu Leu Arg Phe Leu Tyr Thr Gln Leu Glu
            400                 405                 410

ctt tac tta aat aac aaa gat gat caa aaa aga tcc atc ttt cag aaa     1299
Leu Tyr Leu Asn Asn Lys Asp Asp Gln Lys Arg Ser Ile Phe Gln Lys
        415                 420                 425

tca gag cga ggg ggg ttt agg ctg aag gag aat gtc cag ttt cat ctg     1347
Ser Glu Arg Gly Gly Phe Arg Leu Lys Glu Asn Val Gln Phe His Leu
    430                 435                 440

tac atc agc acc tct ccc tgt gga gat gcc aga atc ttc tca cca cat     1395
Tyr Ile Ser Thr Ser Pro Cys Gly Asp Ala Arg Ile Phe Ser Pro His
445                 450                 455                 460

gag cca atc ctg gaa gaa cca gca gat aga cac cca aat cgt aaa gca     1443
Glu Pro Ile Leu Glu Glu Pro Ala Asp Arg His Pro Asn Arg Lys Ala
                465                 470                 475

aga gga cag cta cgg acc aaa ata gag tct ggt gag ggg acg att cca     1491
Arg Gly Gln Leu Arg Thr Lys Ile Glu Ser Gly Glu Gly Thr Ile Pro
            480                 485                 490

gtg cgc tcc aat gcg agc atc caa acg tgg gac ggg gtg ctg caa ggg     1539
Val Arg Ser Asn Ala Ser Ile Gln Thr Trp Asp Gly Val Leu Gln Gly
        495                 500                 505

gag cgg ctg ctc acc atg tcc tgc agt gac aag att gca cgc tgg aac     1587

Glu Arg Leu Leu Thr Met Ser Cys Ser Asp Lys Ile Ala Arg Trp Asn
    510                 515                 520

gtg gtg ggc atc cag gga tcc ctg ctc agc att ttc gtg gag ccc att      1635
Val Val Gly Ile Gln Gly Ser Leu Leu Ser Ile Phe Val Glu Pro Ile
525                 530                 535                 540

tac ttc tcg agc atc atc ctg ggc agc ctt tac cac ggg gac cac ctt      1683
Tyr Phe Ser Ser Ile Ile Leu Gly Ser Leu Tyr His Gly Asp His Leu
                545                 550                 555

tcc agg gcc atg tac cag cgg atc tcc aac ata gag gac ctg cca cct      1731
Ser Arg Ala Met Tyr Gln Arg Ile Ser Asn Ile Glu Asp Leu Pro Pro
            560                 565                 570

ctc tac acc ctc aac aag cct ttg ctc agt ggc atc agc aat gca gaa      1779
Leu Tyr Thr Leu Asn Lys Pro Leu Leu Ser Gly Ile Ser Asn Ala Glu
        575                 580                 585

gca cgg cag cca ggg aag gcc ccc aac ttc agt gtc aac tgg acg gta      1827
Ala Arg Gln Pro Gly Lys Ala Pro Asn Phe Ser Val Asn Trp Thr Val
    590                 595                 600

ggc gac tcc gct att gag gtc atc aac gcc acg act ggg aag gat gag      1875
Gly Asp Ser Ala Ile Glu Val Ile Asn Ala Thr Thr Gly Lys Asp Glu
605                 610                 615                 620

ctg ggc cgc gcg tcc cgc ctg tgt aag cac gcg ttg tac tgt cgc tgg      1923
Leu Gly Arg Ala Ser Arg Leu Cys Lys His Ala Leu Tyr Cys Arg Trp
                625                 630                 635

atg cgt gtg cac ggc aag gtt ccc tcc cac tta cta cgc tcc aag att      1971
Met Arg Val His Gly Lys Val Pro Ser His Leu Leu Arg Ser Lys Ile
            640                 645                 650

acc aag ccc aac gtg tac cat gag tcc aag ctg gcg gca aag gag tac      2019
Thr Lys Pro Asn Val Tyr His Glu Ser Lys Leu Ala Ala Lys Glu Tyr
        655                 660                 665

cag gcc gcc aag gcg cgt ctg ttc aca gcc ttc atc aag gcg ggg ctg      2067
Gln Ala Ala Lys Ala Arg Leu Phe Thr Ala Phe Ile Lys Ala Gly Leu
    670                 675                 680

ggg gcc tgg gtg gag aag ccc acc gag cag gac cag ttc tca ctc acg      2115
Gly Ala Trp Val Glu Lys Pro Thr Glu Gln Asp Gln Phe Ser Leu Thr
685                 690                 695                 700

ccc tgaagatcc                                                        2127
Pro

<210>  9
<211>  701
<212>  PRT
<213>  Homo sapiens

<400>  9

Met Asp Ile Glu Asp Glu Glu Asn Met Ser Ser Ser Ser Thr Asp Val
1               5                   10                  15

Lys Glu Asn Arg Asn Leu Asp Asn Val Ser Pro Lys Asp Gly Ser Thr
                20                  25                  30

Pro Gly Pro Gly Glu Gly Ser Gln Leu Ser Asn Gly Gly Gly Gly Gly
        35                  40                  45

Pro Gly Arg Lys Arg Pro Leu Glu Glu Gly Ser Asn Gly His Ser Lys
    50                  55                  60

Tyr Arg Leu Lys Lys Arg Arg Lys Thr Pro Gly Pro Val Leu Pro Lys
65                  70                  75                  80

Asn Ala Leu Met Gln Leu Asn Glu Ile Lys Pro Gly Leu Gln Tyr Thr
                85                  90                  95

Leu Leu Ser Gln Thr Gly Pro Val His Ala Pro Leu Phe Val Met Ser
            100                 105                 110

Val Glu Val Asn Gly Gln Val Phe Glu Gly Ser Gly Pro Thr Lys Lys
        115                 120                 125

Lys Ala Lys Leu His Ala Ala Glu Lys Ala Leu Arg Ser Phe Val Gln
    130                 135                 140

Phe Pro Asn Ala Ser Glu Ala His Leu Ala Met Gly Arg Thr Leu Ser
145                 150                 155                 160

Val Asn Thr Asp Phe Thr Ser Asp Gln Ala Asp Phe Pro Asp Thr Leu
                165                 170                 175

Phe Asn Gly Phe Glu Thr Pro Asp Lys Ala Glu Pro Pro Phe Tyr Val
            180                 185                 190

Gly Ser Asn Gly Asp Asp Ser Phe Ser Ser Ser Gly Asp Leu Ser Leu
        195                 200                 205

Ser Ala Ser Pro Val Pro Ala Ser Leu Ala Gln Pro Pro Leu Pro Val
    210                 215                 220

Leu Pro Pro Phe Pro Pro Pro Ser Gly Lys Asn Pro Val Met Ile Leu
225                 230                 235                 240

Asn Glu Leu Arg Pro Gly Leu Lys Tyr Asp Phe Leu Ser Glu Ser Gly
                245                 250                 255

Glu Ser His Ala Lys Ser Phe Val Met Ser Val Val Val Asp Gly Gln
            260                 265                 270

Phe Phe Glu Gly Ser Gly Arg Asn Lys Lys Leu Ala Lys Ala Arg Ala
        275                 280                 285

Ala Gln Ser Ala Leu Ala Ala Ile Phe Asn Leu His Leu Asp Gln Thr
    290                 295                 300

Pro Ser Arg Gln Pro Ile Pro Ser Glu Gly Leu Gln Leu His Leu Pro
305                 310                 315                 320

Gln Val Leu Ala Asp Ala Val Ser Arg Leu Val Leu Gly Lys Phe Gly
                325                 330                 335

Asp Leu Thr Asp Asn Phe Ser Ser Pro His Ala Arg Arg Lys Val Leu
            340                 345                 350

Ala Gly Val Val Met Thr Thr Gly Thr Asp Val Lys Asp Ala Lys Val
        355                 360                 365

Ile Ser Val Ser Thr Gly Thr Lys Cys Ile Asn Gly Glu Tyr Met Ser
    370                 375                 380

Asp Arg Gly Leu Ala Leu Asn Asp Cys His Ala Glu Ile Ile Ser Arg
385                 390                 395                 400

Arg Ser Leu Leu Arg Phe Leu Tyr Thr Gln Leu Glu Leu Tyr Leu Asn
                405                 410                 415

Asn Lys Asp Asp Gln Lys Arg Ser Ile Phe Gln Lys Ser Glu Arg Gly
            420                 425                 430

Gly Phe Arg Leu Lys Glu Asn Val Gln Phe His Leu Tyr Ile Ser Thr
        435                 440                 445

Ser Pro Cys Gly Asp Ala Arg Ile Phe Ser Pro His Glu Pro Ile Leu
    450                 455                 460

Glu Glu Pro Ala Asp Arg His Pro Asn Arg Lys Ala Arg Gly Gln Leu
465                 470                 475                 480

Arg Thr Lys Ile Glu Ser Gly Glu Gly Thr Ile Pro Val Arg Ser Asn
                485                 490                 495

Ala Ser Ile Gln Thr Trp Asp Gly Val Leu Gln Gly Glu Arg Leu Leu
            500                 505                 510

Thr Met Ser Cys Ser Asp Lys Ile Ala Arg Trp Asn Val Val Gly Ile
        515                 520                 525

Gln Gly Ser Leu Leu Ser Ile Phe Val Glu Pro Ile Tyr Phe Ser Ser
    530                 535                 540

Ile Ile Leu Gly Ser Leu Tyr His Gly Asp His Leu Ser Arg Ala Met
545                 550                 555                 560

Tyr Gln Arg Ile Ser Asn Ile Glu Asp Leu Pro Pro Leu Tyr Thr Leu
                565                 570                 575

Asn Lys Pro Leu Leu Ser Gly Ile Ser Asn Ala Glu Ala Arg Gln Pro
            580                 585                 590

Gly Lys Ala Pro Asn Phe Ser Val Asn Trp Thr Val Gly Asp Ser Ala
        595                 600                 605

Ile Glu Val Ile Asn Ala Thr Thr Gly Lys Asp Glu Leu Gly Arg Ala
    610                 615                 620

Ser Arg Leu Cys Lys His Ala Leu Tyr Cys Arg Trp Met Arg Val His
625                 630                 635                 640

Gly Lys Val Pro Ser His Leu Leu Arg Ser Lys Ile Thr Lys Pro Asn
                645                 650                 655

Val Tyr His Glu Ser Lys Leu Ala Ala Lys Glu Tyr Gln Ala Ala Lys
            660                 665                 670

Ala Arg Leu Phe Thr Ala Phe Ile Lys Ala Gly Leu Gly Ala Trp Val
        675                 680                 685

Glu Lys Pro Thr Glu Gln Asp Gln Phe Ser Leu Thr Pro
    690                 695                 700

<210>  10
<211>  27
<212>  DNA
<213>  Artificial Sequence

<220>
<223>  Synthetic DNA

<400>  10
gtgctcgctt cggcagcaca tatacta                                          27

<210>  11
<211>  20
<212>  DNA
<213>  Artificial Sequence

<220>
<223>  Synthetic DNA

<400>  11
gcggacttcg gtccgctttt                                                   20


<210>  12
<211>  216
<212>  DNA
<213>  Artificial Sequence

<220>
<223>  Synthetic DNA

<400>  12
atacttacct ggcaggggag ataccatgat cacgaaggtg gttttcccag ggcgaggctt       60

atccattgca ctccggatgt gctgacccct gcgatttccc caaatgtggg aaactcgact      120

gcataatttg tggtagtggg ggactgcgtt cgcgctttcc cctgactttc tggagtttca      180

aaagtagact gtacgctaag ggtcatatct tttttt                                216


<210>  13
<211>  149
<212>  DNA
<213>  Artificial Sequence

<220>
<223>  Synthetic DNA

<400>  13
aattttggga gcaggttttc tgacttcggt cggaaaaccc ctcccaattt cactggtcta       60

caatgaaagc aaaacagttc tcttccccgc tccccggtgt gtgagagggg ctttgatcct      120

tctctggttt cctaggaaac gcgtatgtg                                        149


<210>  14
<211>  118
<212>  DNA
<213>  Artificial Sequence

<220>
<223>  Synthetic DNA

<400>  14
gtctacggcc ataccaccct gaacgcgccc gatctcgtct gatctcggaa gctaagcagg       60

gtcgggcctg gttagtactt ggatgggaga ccgcctggga ataccgggtg ctgtaggc        118


<210>  15
<211>  24
<212>  DNA
<213>  Artificial Sequence

<220>
<223>  Synthetic DNA

<400>  15
gggtttgggt ttgggtttgg gttt                                              24


<210>  16
<211>  24
<212>  DNA
<213>  Artificial Sequence

<220>
<223>  Synthetic DNA

<400>  16
agagggccct gaagagggcc cttt                                              24


<210>  17
<211>  241
<212>  DNA
<213>  Artificial Sequence

<220>
<223>  Synthetic DNA

<400>  17
gagggcctat ttcccatgat tccttcatat ttgcatatac gatacaaggc tgttagagag      60

ataattagaa ttaatttgac tgtaaacaca aagatattag tacaaaatac gtgacgtaga     120

aagtaataat ttcttgggta gtttgcagtt ttaaaattat gttttaaaat ggactatcat     180

atgcttaccg taacttgaaa gtatttcgat ttcttggctt tatatatctt gtggaaagga     240

c                                                                     241


<210>  18
<211>  92
<212>  DNA
<213>  Artificial Sequence

<220>
<223>  Artifitial DNA

<400>  18
ggggtgctcg cttcggcagc acatatacta gtcgacgaag gttcagcagc tcgaaccaag      60

tctagagcgg acttcggtcc gctttttttt tt                                    92


<210>  19
<211>  400
<212>  DNA
<213>  Artificial Sequence

<220>
<223>  Artifitial DNA

<400>  19
acacaggcta aggaccagct tctttgggag agaacagacg caggggcggg agggaaaaag      60

ggagaggcag acgtcacttc cccttggcgg ctctggcagc agattggtcg gttgagtggc     120

agaaaggcag acggggactg ggcaaggcac tgtcggtgac atcacggaca gggcgacttc 180

tatgtagatg aggcagcgca gaggctgctg cttcgccact tgctgcttca ccacgaagga 240

gttcccgtgc cctgggagcg ggttcaggac cgctgatcgg aagtgagaat cccagctgtg 300

tgtcagggct ggaaagggct cgggagtgcg cggggcaagt gaccgtgtgt gtaaagagtg 360

aggcgtatga ggctgtgtcg gggcagaggc ccaagatctc 400

<210> 20
<211> 270
<212> DNA
<213> Artificial Sequence

<220>
<223> Artifitial DNA

<400> 20
gtttcaggat cctaacaaca taggagctgt gattggctgt tttcagccaa tcagcactga 60

ctcatttgca tagcctttac aagcggtcac aaactcaaga aacgagcggt tttaatagtc 120

ttttagaata ttgtttatcg aaccgaataa ggaactgtgc tttgtgattc acatatcagt 180

ggaggggtgt ggaaatggca ccttgatctc accctcatcg aaagtggagt tgatgtcctt 240

ccctggctcg ctacagacgc acttccgcaa 270

<210> 21
<211> 85
<212> DNA
<213> Artificial Sequence

<220>
<223> Artifitial DNA

<400> 21
ccccgggctg gcggtgtcgg ctgcaatccg gcgggcacgg ccgggccggg ctgggctctt 60

ggggcagcca ggcgcctcct tcagc 85

<210> 22
<211> 233
<212> DNA
<213> Artificial Sequence

<220>
<223> Artifitial DNA

<400> 22
atgaaggttc agcagctcga accaagggca ggggagatac catgatcacg aaggtggttt 60

tcccagggcg aggcttatcc attgcactcc ggatgtgctg acccctgcga tttccccaaa 120

tgtgggaaac tcgactgcat aatttgtggt agtgggggac tgcgttcgcg ctttcccctg 180

actttctgga gtttcaaaag tagactgtac gctaagggtc atatcttttt ttt 233

<210>  23
<211>  173
<212>  DNA
<213>  Artificial Sequence

<220>
<223>  Artifitial DNA

<400>  23
gaaggttcag cagctcgaac caagaatttt tggagcaggt tttctgactt cggtcggaaa      60

acccctccca atttcactgg tctacaatga aagcaaaaca gttctcttcc ccgctccccg      120

gtgtgtgaga ggggctttga tccttctctg gtttcctagg aaacgcgtat gtg      173


<210>  24
<211>  176
<212>  DNA
<213>  Artificial Sequence

<220>
<223>  Synthetic DNA

<400>  24
gtctacggcc ataccaccct gaacgcgccc gatctcgtct gatctcggaa gctaagcagg      60

gtcgggcctg gttagtactt ggatgggaga ccgcctggga ataccgggtg ctgtaggcgt      120

cgacgaaggt tcagcagctc gaaccaagtc tagagcggac ttcggtccgc tttttt      176


<210>  25
<211>  57
<212>  DNA
<213>  Artificial Sequence

<220>
<223>  Synthetic DNA

<400>  25
ggggaaggtt cagcagctcg aaccaagggg tttgggtttg ggtttgggtt ttttttt      57


<210>  26
<211>  57
<212>  DNA
<213>  Artificial Sequence

<220>
<223>  Synthetic DNA

<400>  26
ggggggtttg ggtttgggtt tgggtttgaa ggttcagcag ctcgaaccaa gtttttt      57


<210>  27
<211>  60
<212>  DNA
<213>  Artificial Sequence

<220>
<223>  Synthetic DNA

<400>  27
ggggaaggtt cagcagctcg aaccaagtct agagggccct gaagagggcc cttttttttt        60


<210>  28
<211>  60
<212>  DNA
<213>  Artificial Sequence

<220>
<223>  Synthetic DNA

<400>  28
gggagagggc cctgaagagg gcccttttct gaaggttcag cagctcgaac caagtttttt        60


<210>  29
<211>  85
<212>  DNA
<213>  Artificial Sequence

<220>
<223>  Synthetic DNA

<400>  29
ggggggtttg ggtttgggtt tgggtttgaa ggttcagcag ctcgaaccaa gtctagaggg        60

ccctgaagag ggcccttttt ttttt                                              85


<210>  30
<211>  85
<212>  DNA
<213>  Artificial Sequence

<220>
<223>  Synthetic DNA

<400>  30
gggagagggc cctgaagagg gcccttttct gaaggttcag cagctcgaac caaggggttt        60

gggtttgggt ttgggttttt ttttt                                              85


<210>  31
<211>  84
<212>  DNA
<213>  Artificial Sequence

<220>
<223>  Synthetic DNA

<400>  31
ggggaaggtt cagcagctcg aaccaagggg tttgggtttg ggtttgggtt ttctagaggg        60

ccctgaagag ggcccttttt tttt                                               84


<210>  32
<211>  82
<212>  DNA
<213>  Artificial Sequence

<220>
<223>  Synthetic DNA

<400>  32
ggggtttggg tttgggtttg ggtttagagg gccctgaaga gggccctttt ctgaaggttc      60

agcagctcga accaagtttt tt                                              82


<210>  33
<211>  33
<212>  DNA
<213>  Artificial Sequence

<220>
<223>  Synthetic DNA

<400>  33
ggggaaggtt cagcagctcg aaccaagttt ttt                                  33


<210>  34
<211>  516
<212>  DNA
<213>  Artificial Sequence

<220>
<223>  Synthetic DNA

<400>  34
cacgtcgtgc ctcacatcga gcccgtggct agatgcatca tccctgatct gatcggaatg      60

ggtaagtccg gcaagagcgg gaatggctca tatcgcctcc tggatcacta caagtacctc     120

accgcttagt tcgagctgct gaaccttcca aagaaaatca tctttgtggg ccacgactgg     180

ggggcttgtc tggcctttca ctactcctac gagcaccaag acaagatcaa ggccatcgtc     240

catgctgaga gtgtcgtgga cgtgatcgag tcctgggacg agtggcctga catcgaggag     300

gatatcgccc tgatcaagag cgaagagggc gagaaaatgg tgcttgagaa taacttcttc     360

gtcgagacca tgctcccaag caagatcatg cggaaactgg agcctgagga gttcgctgcc     420

tacctggagc cattcaagga gaagggcgag gttagacggc ctaccctctc ctggcctcgc     480

gagatccctc tcgttaaggg aggcaagccc gacgtc                              516


<210>  35
<211>  22
<212>  DNA
<213>  Artificial Sequence

<220>
<223>  Synthetic DNA

<400>  35
atggcttcca aggtgtacga cc                                              22


<210>  36

<211>  22
<212>  DNA
<213>  Artificial Sequence

<220>
<223>  Synthetic DNA

<400>  36
ttactgctcg ttcttcagca cg                                                22


<210>  37
<211>  20
<212>  DNA
<213>  Artificial Sequence

<220>
<223>  Synthetic DNA

<400>  37
ctcgctgcaa gcaaatgaac                                                   20


<210>  38
<211>  20
<212>  DNA
<213>  Artificial Sequence

<220>
<223>  Synthetic DNA

<400>  38
tcgtcccagg actcgatcac                                                   20


<210>  39
<211>  92
<212>  DNA
<213>  Artificial Sequence

<220>
<223>  Synthetic DNA

<400>  39
ggggtgctcg cttcggcagc acatatacta gtcgacggcg cacagacatc tcgaagcact      60

tctagagcgg acttcggtcc gctttttttt tt                                     92


<210>  40
<211>  56
<212>  DNA
<213>  Artificial sequence

<220>
<223>  Synthetic DNA

<400>  40
ggggcgcaca gacatctcga agcactgggt ttgggtttgg gtttgggttt tttttt          56


<210>  41
<211>  55

<212>  DNA
<213>  Artificial sequence

<220>
<223>  Synthetic DNA

<400>  41
ggggtttggg tttgggtttg ggtttggcgc acagacatct cgaagcactt ttttt          55


<210>  42
<211>  59
<212>  DNA
<213>  Artificial sequence

<220>
<223>  Synthetic DNA

<400>  42
ggggcgcaca gacatctcga agcacttcta gagggccctg aagagggccc ttttttttt     59


<210>  43
<211>  60
<212>  DNA
<213>  Artificial sequence

<220>
<223>  Synthetic DNA

<400>  43
gggagagggc cctgaagagg gccctttctt ggcgcacaga catctcgaag cacttttttt    60


<210>  44
<211>  83
<212>  DNA
<213>  Artificial sequence

<220>
<223>  Synthetic DNA

<400>  44
ggggtttggg tttgggtttg ggtttggcgc acagacatct cgaagcactt ctagagggcc    60

ctgaagaggg cccttttttt ttt                                            83


<210>  45
<211>  85
<212>  DNA
<213>  Artificial sequence

<220>
<223>  Synthetic DNA

<400>  45
gggagagggc cctgaagagg gccctttctt ggcgcacaga catctcgaag cactgggttt    60

gggtttgggt ttgggttttt ttttt                                          85


<210>  46

<211>  83
<212>  DNA
<213>  Artificial sequence

<220>
<223>  Synthetic DNA

<400>  46
ggggcgcaca gacatctcga agcactgggt ttgggtttgg gtttgggttt tctagagggc      60

cctgaagagg gccctttttt ttt                                              83


<210>  47
<211>  82
<212>  DNA
<213>  Artificial sequence

<220>
<223>  Synthetic DNA

<400>  47
ggggtttggg tttgggtttg ggtttagagg gccctgaaga gggccctttt ctggcgcaca      60

gacatctcga agcacttttt tt                                               82


<210>  48
<211>  32
<212>  DNA
<213>  Artificial sequence

<220>
<223>  Synthetic DNA

<400>  48
ggggcgcaca gacatctcga agcacttttt tt                                    32


<210>  49
<211>  19
<212>  DNA
<213>  Artificial sequence

<220>
<223>  Synthetic DNA

<400>  49
acccgcttaa aagcttggc                                                   19


<210>  50
<211>  20
<212>  DNA
<213>  Artificial sequence

<220>
<223>  Synthetic DNA

<400>  50
ccacggtagg ctgagaaatg                                                  20

<210>  51
<211>  20
<212>  DNA
<213>  Artificial sequence

<220>
<223>  Synthetic DNA

<400>  51
catgctgttc agcagctcgc                                                    20


<210>  52
<211>  24
<212>  DNA
<213>  Artificial sequence

<220>
<223>  Synthetic DNA

<400>  52
gaaggttcag cagctcgaac caag                                               24

## Description

### TECHNICAL FIELD

The present invention relates to a guide RNA for editing a target RNA by guiding ADAR, particularly an antisense guide RNA with an added functional region.

### BACKGROUND ART

There is an RNA editing mechanism by a single nucleotide mutation in vivo. In particular, RNA editing from A to I, in which adenosine is converted to inosine by deamination of adenosine, is most observed, and the number of RNAs to be targeted is as high as 4 million. ADAR (adenosine deaminase acting on RNA) is known as an enzyme that converts adenosine to inosine using a double-stranded RNA as a substrate. ADAR has three subtypes (ADAR1, ADAR2, and ADAR3) with different genes, and ADAR has a double-stranded RNA-binding domain at the N terminus and a deaminase domain at the C terminus. ADAR2 highly efficiently edits a subunit GluR2 that constitutes a 3-amino-3-hydroxy-5-methyl-4-isooxazole propionic acid (AMPA) glutamate receptor especially in the central nervous system (Nature, 1996, Vol. 379, pp. 460-464). Since inosine converted from adenosine has a structure similar to that of guanosine, it is recognized as guanosine at the translation stage, and as a result, an amino acid mutation may sometimes be introduced into the coding region. A retrotransposon (FEBS Letters, 2006, Vol. 580, pp. 2301-2305) and a microRNA (miRNA) precursor have also been reported as substrates for ADAR in the non-coding region, and it has been suggested that ADAR may be responsible for various functions in vivo (Annu. Rev. Biochem., 2010, Vol. 79, pp. 321-349).

A variant of ADAR with an artificially introduced mutation has also been reported. For example, a variant of ADAR that can recognize not only adenosine but also cytidine by introducing a mutation into ADAR, and can also perform RNA editing from C to U, in which cytidine is changed to uridine by deamination of cytidine has been reported. (Science, 2019, Vol. 365, pp. 382-386).

In recent years, as RNA editing using wild-type ADAR and an artificial guide RNA, several guide RNAs for editing a target RNA containing a GluR2-derived ADAR-recruiting base sequence have been reported (WO 2016/097212, WO 2017/050306, WO 2017/010556, WO 2019/111957, Nucleic Acids Research, 2017, Vol. 45, pp. 2797-2808, Nature Methods, 2019, Vol. 16, pp. 239-242).

In addition, as an RNA editing technique using an antisense oligonucleotide, which is an artificially synthesized nucleic acid, as a guide RNA, a technique for editing a target RNA by using an antisense oligonucleotide of 35 or more bases complementary to an mRNA containing adenosine to be targeted for editing, and forming a double strand having an incomplete helical structure with the mRNA and recruiting ADAR has also been reported (PTLs 1 to 3). Further, it has also been reported that editing of a target RNA by ADAR can be induced using an artificial RNA of 100 or more bases (NPL 1). In addition, editing of a target RNA by a fusion protein of ADAR1 deaminase domain-MS2 coat protein (MCP) and an antisense-MS2 RNA conjugate system has also been reported (NPL 2).

Thus, there are several reports on RNA editing using an antisense oligonucleotide. However, in order to use a target RNA editing guide RNA as a pharmaceutical product, it is necessary for the guide RNA to show a given editing efficiency in a cell, but an RNA editing technique having a high editing efficiency has not been sufficiently studied.

On the other hand, RNA sequences having various functions such as a small nuclear RNA (snRNA), a ribosomal RNA (rRNA), an RNA having a higher-order structure such as a guanine quadruplex structure or a stem-loop structure are known, and it has also been reported that some of them are added to an RNA such as a guide RNA or an antisense oligonucleotide.

In a eukaryote, an snRNA acts on splicing or processing of a precursor messenger RNA (pre-mRNA) via a ribonucleoprotein (RNP). In particular, 5 types of snRNPs (U1, U2, U4, U5, and U6) constitute a spliceosome involved in splicing, and the snRNAs are abundantly and stably expressed in almost all eukaryotic cells (Biological Chemistry, 2018, Vol. 399, pp. 1265-1276).

U6 snRNA is stably and abundantly expressed in all human cells and forms a ribonucleoprotein U6 snRNP in the nucleus. U6 snRNP constitutes a spliceosome and controls splicing in an RNA precursor. The transcription initiation element upstream of a U6 snRNA gene is known as a potent polymerase III (polIII) promoter that expresses a given RNA (a ribozyme, an antisense ribonucleic acid, an RNA aptamer, or the like) (NPL 3). NPLs 3 to 5 report that in a small RNA expression cassette composed of a sequence encoding U6 (hereinafter, an expression cassette containing a sequence encoding U6 described in NPLs 3 to 5 is referred to as "U6 cassette"), the higher-order structure of the U6 sequence functions to localize the inserted RNA in the nucleus. The U6 cassette contains an artificial stem-loop sequence immediately upstream of a poly(U) terminator sequence as a device for stably and strongly expressing a small RNA. An artificial stem-loop sequence containing a tetraloop formed of an RNA sequence UNCG has a most thermodynamically stable stem-loop structure (Nucleic Acids Research, 1991, Vol. 19, pp. 5901-5905). It is suggested that the stable stem-loop structure shows resistance to 3'-5' exonuclease activity and stabilizes the transcript of the U6 cassette (NPLs 3 to 5).

U1 snRNA is composed of a sequence complementary to a splicing donor sequence (junction of exon 3' and intron 5') and a sequence having a clover-like structure. It is known that chimeric U1 snRNA in which the splicing donor sequence of U1 snRNA has been substituted with an antisense sequence of a target gene can induce exon skipping (Mol. Ther., 2010, Vol. 18, pp. 1675-1682, Proc. Nat. Acad. Sci., 2002, Vol. 99, pp. 9456-9461). A sequence of 400 bases upstream of a U1 snRNA gene contains a polII promoter and is also used for transcription of chimeric U1 snRNA (Mol. Ther., 2004, Vol. 10, pp. 191-199).

U7 snRNA is responsible for the processing of a histone pre-mRNA. U7 snRNA is composed of a sequence complementary to a histone 3 pre-mRNA, an Sm protein binding site, and a stem-loop sequence. By adding a mutation, the Sm protein binding site becomes capable of binding to D1 and D2 proteins that form a spliceosome instead of the original Lsm protein (Cell. Mol. Life Sci., 2004, Vol. 61, pp. 2560-2570). It has been reported that a mutant chimeric U7 snRNA composed of an antisense sequence of a target gene, a mutant Sm protein binding site, and a stem-loop sequence can induce exon skipping (Nat. Med., 2014, Vol. 20, pp. 992-1000). A sequence of 270 bases upstream of a U7 snRNA gene contains a polymerase II (polII) promoter and is also used for transcription of the mutant chimeric U7 snRNA (Science, 2004, Vol. 306, pp. 1796-1799).

A ribosome is a cell organelle that synthesizes a protein in a cell and is composed of two subunits. The large subunit of a eukaryote is composed of 28S, 5.8S, and 5S rRNAs and various proteins, which are assembled in the nucleolus and then transported to the cytoplasm. 5S rRNAhas a secondary structure composed of 5 helices and 5 loops. NPL 5 reports that when an antisense sequence linked to 5S rRNA is transcribed from a polIII promoter upstream of a 5S gene using an expression cassette of an antisense sequence containing a 5S rRNA sequence, it is localized in the cytoplasm (hereinafter, the expression cassette containing 5S rRNA described in NPL 5 is referred to as "5S cassette"). Similarly to the U6 cassette, the 5S cassette has an artificial stem-loop sequence immediately upstream of the terminator sequence.

The guanine quadruplex (Gq: G-quadruplex) structure is a repeating sequence containing guanine, which is known as a thermodynamically stable DNA or RNA higher-order structure in the in vivo environment. In the formation of the higher-order structure, a monovalent cation is needed. Gq was originally reported as a sequence found in a telomere (Cell, 1989, Vol. 59, pp. 871-880). Known functions of Gq include transcriptional translation, epigenome regulation, and the like (EMBO Reports, 2015, Vol. 16, pp. 910-922). It has been reported that for a Cas9 guide RNA, the genome editing efficiency is improved by adding Gq to the 3' end (NPL 6). Further, it has been reported that the efficiency of gene expression suppression by an RNA degradation enzyme is increased by adding Gq to an antisense oligonucleotide (PTL 4).

The stem-loop structure, which is a higher-order structure of a nucleic acid found in a small RNA, is known to contribute to a translational regulation function (Cell. Mol. Life Sci., 2006, Vol. 63, pp. 901-908). A BoxB sequence derived from λ phage has a stem-loop structure and shows a strong affinity for λN protein (Biol. Cell. 2008, Vol. 100, pp. 125-138, J. Am. Chem. Soc., 2002, Vol. 124, pp. 10966-10967). It has been reported that in order to recruit ADAR to a guide RNA by utilizing this property, a BoxB sequence is added to the guide RNA and λN protein is fused to the ADAR (NPL 7). Further, PTL 5 describes that in order to stabilize a guide RNA designed based on the sequence of GluR2, a BoxB sequence can be added to the 3' end of the guide RNA, but NPL 8 describes that the effect of stabilization by the addition of the BoxB sequence to the guide RNA was small.

As described above, RNA sequences having various functions have been reported, but it has not been reported that an RNA sequence having a function is added in order to contribute to the editing activity of a guide RNA that edits a target RNA.

### CITATION LIST

### PATENT LITERATURE

PTL 1: WO 2017/220751
PTL 2: WO 2018/041973
PTL 3: WO 2018/134301
PTL 4: WO 2017/188898
PTL 5: WO 2017/050306

### NON PATENT LITERATURE

NPL 1: Nat. Biotechnol., 2019, Vol. 37, pp. 1059-1069
NPL 2: Protein Eng. Des. Sel., 2018, Vol. 1, pp. 471-478
NPL 3: Gene Ther., 1997, Vol. 4, pp. 45-54
NPL 4: Nat. Biotechnol., 2002, Vol. 20, pp. 505-508
NPL 5: Mol. Ther., 2003, Vol. 7, pp. 237-247
NPL 6: Chem. Commun., 2018, Vol. 54, pp. 2377-2380
NPL 7: Nucleic Acids Research, 2016, Vol. 44, p. e157
NPL 8: Nucleic Acids Research, 2017, Vol. 45, pp. 2797-2808

### SUMMARY OF INVENTION

### TECHNICAL PROBLEM

An antisense guide RNA for editing a target RNA by ADAR and a nucleic acid encoding the same are provided.

### SOLUTION TO PROBLEM

As a result of intensive studies on a guide RNA for editing a target RNA by guiding ADAR, the present inventors found that a guide RNA that contains an antisense region to which at least one functional region is linked, and does not substantially contain an ADAR-recruiting base sequence shows a high editing efficiency in a cell, and thus completed the present invention.

That is, the present invention relates to the following [1] to [23].
[1] A guide RNA for editing a target RNA by ADAR, containing at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence.
[2] The guide RNA according to [1], wherein the at least one functional region is directly bound to the antisense region or linked thereto via a linker.
[3] The guide RNA according to [1] or [2], wherein the antisense region has a base to form a mismatched base pair with the target RNA.
[4] The guide RNA according to any one of [1] to [3], wherein the functional region is a region composed of a base sequence having any one or more functions among functions including stabilization of the guide RNA, localization of the guide RNA to the nucleus, localization of the guide RNA to the cytoplasm, promotion of double strand formation between the target RNA and the antisense region, inhibition of non-specific double strand formation by the antisense region, and stabilization of a complex formed of the target RNA and the antisense region.
[5] The guide RNA according to any one of [1] to [4], wherein the functional region is a region composed of an snRNA sequence, a region composed of an rRNA sequence, a region composed of a base sequence to form a guanine quadruplex structure, or a region composed of a base sequence to form a stem-loop structure, or any combination thereof.
[6] The guide RNA according to [5], wherein the functional region is a region composed of an snRNA sequence and an optionally contained region composed of a base sequence to form a stem-loop structure.
[7] The guide RNA according to [6], wherein the region composed of an snRNA sequence is a region composed of a U6 snRNA sequence, a region composed of a U1 snRNA sequence, or a region composed of a U7 snRNA sequence.
[8] The guide RNA according to [5], wherein the functional region is a region composed of an rRNA sequence and an optionally contained region composed of a base sequence to form a stem-loop structure.
[9] The guide RNA according to [8], wherein the region composed of an rRNA sequence is a region composed of a 5S rRNA sequence.
[10] The guide RNA according to [5], wherein the functional region is a region composed of a base sequence to form a guanine quadruplex structure, or a region composed of a base sequence to form a stem-loop structure, or a combination thereof.
[11] The guide RNA according to any one of [1] to [5], wherein the guide RNA is composed of an antisense region, a functional region composed of a base sequence to form a guanine quadruplex structure, an optionally contained additional functional region, and an optionally contained linker, and the antisense region, the functional region composed of a base sequence to form a guanine quadruplex structure, and the optionally contained additional functional region are linked in this order from the 5' side to the 3' side.
[12] The guide RNA according to any one of [1] to [5], wherein the guide RNA is composed of a functional region composed of a base sequence to form a stem-loop structure, an antisense region, an optionally contained additional functional region, and an optionally contained linker, and the functional region composed of a base sequence to form a stem-loop structure and the antisense region are linked in this order from the 5' side to the 3' side.
[13] A system for editing a target RNA, including the guide RNA according to any one of [1] to [12] and ADAR.
[14] A nucleic acid, encoding the guide RNA according to any one of [1] to [12].
[15] An expression vector, containing a nucleic acid encoding the guide RNA according to any one of [1] to [12].
[16] The expression vector according to [15], further containing a nucleic acid encoding ADAR.
[17] The expression vector according to [16], wherein the ADAR is ADAR1 or ADAR2
[18] A host cell into which the expression vector according to any one of [15] to [17] has been introduced.
[19] A method for producing an expression vector, including a step of culturing the host cell according to [18].
[20] A pharmaceutical composition, containing the expression vector according to any one of [15] to [17] and a pharmaceutically acceptable excipient.
[21] A pharmaceutical composition, containing the expression vector according to [15], an expression vector containing a nucleic acid encoding ADAR, and a pharmaceutically acceptable excipient.
[22] A method for editing a target RNA, including a step of introducing a nucleic acid encoding the guide RNA according to any one of [1] to [12] into a cell, a virus, or the like.
[23] A polynucleotide for editing a target RNA by ADAR, containing at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence.

### ADVANTAGEOUS EFFECTS OF INVENTION

An antisense guide RNA with an added functional region can be used as a guide RNA for editing a target RNA.

### DESCRIPTION OF EMBODIMENTS

The present invention will be described in detail below, but the present invention is not limited thereto. If the term used herein is not specifically defined, it will be used in a sense generally accepted by those skilled in the art.

### <Guide RNA of Present Invention>

The present invention provides a guide RNA for editing a target RNA by ADAR, which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence (hereinafter, also referred to as "the guide RNA of the present invention"). In the present invention, the "guide RNA for editing a target RNA by ADAR" refers to an RNA molecule that has a region which is complementary to a portion of the target RNA and forms a double strand with the target RNA, and that guides ADAR to the target RNA.

### 1. Target RNA

In the present invention, the "target RNA" refers to an RNA targeted for editing by the guide RNA of the present invention. In a portion of the target RNA, adenosine to be converted to inosine, or cytidine to be converted to uridine is contained. In some embodiments, the target RNA is any RNA sequence, in which adenosine is contained and the cell function and/or gene expression can be controlled by conversion of the adenosine to inosine. In other embodiments, the target RNA is any RNA sequence, in which cytidine is contained and the cell function and/or gene expression can be controlled by conversion of the cytidine to uridine. Here, the phrase "the cell function can be controlled" means that due to a change in the function of an RNA, the translation thereof, and the function of a protein in a cell having a target RNA, the function of the cell can be controlled. The phrase "gene expression can be controlled" means that the expression of a gene having a target RNA can be controlled. The target RNA can be present in an RNA containing an exon, an intron, a coding region or various untranslated regions in an exon (for example, a retrotransposon, a microRNA (miRNA), a transfer RNA (tRNA), a ribosomal RNA (rRNA), etc.). The target RNA can be present in an RNA contained in a eukaryotic cell, a mammalian cell, a virus, a prokaryotic cell, a bacterium, a phage, or the like.

### 2. Antisense Region

The guide RNA of the present invention contains an antisense region. In the present invention, the "antisense region" refers to a region having a base sequence complementary to a portion of the target RNA and composed of a sequence to form a double strand with the target RNA. The length of the base sequence constituting the antisense region is, in some embodiments, 10 bases to 100 bases, 10 bases to 80 bases, 10 bases to 60 bases, or 10 bases to 40 bases, in some embodiments, 15 bases to 40 bases, in some embodiments, 10 bases to 38 bases, in some embodiments, 15 to 38 bases, and in some embodiments, 18 to 38 bases. In some embodiments, the antisense region may contain a base to form a mismatched base pair or a base to form a wobble base pair with the target RNA. In some embodiments, the antisense region may contain a base to form a mismatched base pair with the target RNA.

The "mismatched base pair" as used herein is a G-A, C-A, U-C, A-A, G-G, C-C, or U-U base pair. The " wobble base pair" as used herein is a G-U, I-U, I-A, or I-C base pair. In some embodiments, the antisense region is a region composed of a base sequence which has a base to form a mismatched base pair with adenosine or cytidine contained in a portion of the target RNA, and forms a double strand with the target RNA. In some embodiments, the antisense region is a region composed of a base sequence which has a base to form a mismatched base pair with adenosine contained in a portion of the target RNA, and forms a double strand with the target RNA. In some embodiments, the antisense region is a region composed of a base sequence which has cytidine being a base to form a mismatched base pair with adenosine contained in a portion of the target RNA, and forms a double strand with the target RNA.

The base sequence constituting the antisense region can be appropriately designed by those skilled in the art in consideration of the sequence of the target RNA, the base length, the position of the mismatched base formed with the target RNA, the off-target effect, or the like. In order to improve the editing efficiency of the target RNA by ADAR to adenosine or cytidine, the base sequence constituting the antisense region can also be designed so as to form a mismatched base pair or a wobble base pair with the adenosine or cytidine (RNA, 2001, Vol. 7, pp. 846-858, Nat. Biotechnol., 2019, Vol. 37, pp. 1059-1069).

The "ASR" as used herein denotes the antisense region.

### 3. Functional Region

The guide RNA of the present invention contains at least one functional region. In the present invention, the "functional region" refers to a region composed of a base sequence having any one or more functions among functions including stabilization of the guide RNA, localization of the guide RNA to the nucleus, localization of the guide RNA to the cytoplasm, promotion of double strand formation between the target RNA and the antisense region, inhibition of non-specific double strand formation by the antisense region, stabilization of a complex formed of the target RNA and the antisense region, and the like. In some embodiments, the functional region is a region composed of a base sequence having any one or more functions among functions including stabilization of the guide RNA, localization of the guide RNA to the nucleus, localization of the guide RNA to the cytoplasm, promotion of double strand formation between the target RNA and the antisense region, inhibition of non-specific double strand formation by the antisense region, and stabilization of a complex formed of the target RNA and the antisense region.

In some embodiments, the guide RNA of the present invention contains, as the functional region, (a) a region composed of a base sequence that promotes stabilization of the guide RNA, (b) a region composed of a base sequence that promotes localization of the guide RNA to the nucleus, (c) a region composed of a base sequence that promotes localization of the guide RNA to the cytoplasm, (d) a region composed of a base sequence that promotes double strand formation between the target RNA and the antisense region, (e) a region composed of a base sequence that inhibits non-specific double strand formation by the antisense region, or (f) a region composed of a base sequence that promotes stabilization of a complex formed of the target RNA and the antisense region, or a region composed of a base sequence having any two or more functions among the functions described in the above (a) to (f). The expression "the functional region has one or more functions among these" is sometimes collectively referred to as "having a function". In addition, the expression "having the functions described in (a), (b), and (d)" is sometimes collectively referred to as "having the function of an snRNA sequence". The expression "having the functions described in (a) and (c)" is sometimes collectively referred to as "having the function of an rRNA sequence".

In the present description, the "promoting stabilization of the guide RNA" refers to imparting resistance to an RNA degradation enzyme. The function can be evaluated by measuring the residual amount of the guide RNA in the presence of an RNA degradation enzyme, the residual amount of the guide RNA in a cell under transcription inhibition after introduction of a nucleic acid encoding the guide RNA into the cell, or the like by a known method. For example, it can be evaluated by the method described in Example 10. The "promoting localization of the guide RNA to the nucleus" refers to promoting transfer and localization of the guide RNA introduced into a cell to the nucleus. The function can be evaluated by detecting the intracellular distribution or amount of the guide RNA by a known method. For example, it can be confirmed by in situ hybridization (Mol. Ther., 2003, Vol. 7, pp. 237-247). The "promoting localization of the guide RNA to the cytoplasm" refers to promoting retention of the guide RNA introduced into a cell in the cytoplasm. The function can be evaluated by detecting the intracellular distribution or amount of the guide RNA by a known method. For example, it can be confirmed by in situ hybridization (Mol. Ther., 2003, Vol. 7, pp. 237-247). The "promoting double strand formation between the target RNA and the antisense region" refers to improving affinity between the target RNA and the antisense region. The function can be evaluated by an affinity assay using a known method (BMC Botech. 2008, Vol. 8, article number 48). The "inhibiting non-specific double strand formation by the antisense region" refers to reducing non-specific double strand formation (hereinafter, also referred to as "off-target effect"). The function can be evaluated by a known method such as RNA sequencing (Nat. Biotechnol., 2019, Vol. 37, pp. 657-666). The "promoting stabilization of a complex formed of the target RNA and the antisense region" refers to maintaining the state of a complex containing a double strand formed of the target RNA and the antisense region. The function can be evaluated by measuring the residual amount of a double strand formed of the target RNA and the antisense region in the presence of an RNA degradation enzyme that degrades an RNA of a DNA/RNA hybrid strand by a known method. For example, it can be evaluated by measuring the residual amount of a double strand formed of the target RNA and the guide RNA composed of a DNA sequence in the presence of RNase H (Antiviral Chemistry & Chemotherapy, 1996, Vol. 7, pp. 86-93).

In some embodiments, the region composed of a base sequence that promotes stabilization of the guide RNA is a region composed of a base sequence to form a thermodynamically stabilized higher-order structure. The thermodynamically stabilized higher-order structure is not limited as long as it is a structure that shows resistance to nuclease activity. In some embodiments, the region composed of a base sequence that promotes stabilization of the guide RNA is a region composed of a small nuclear RNA (snRNA) sequence, a region composed of a ribosomal RNA (rRNA) sequence, a region composed of a base sequence to form a duplex structure, a region composed of a base sequence to form a triplex structure, a region composed of a base sequence to form a quadruplex structure, a region composed of a base sequence to form a stem-loop structure, or the like. In some embodiments, the region composed of a base sequence that promotes stabilization of the guide RNA is a region composed of an snRNA sequence, a region composed of a base sequence to form a guanine quadruplex (Gq: G-quadruplex) structure, or a region composed of a base sequence to form a stem-loop structure.

In some embodiments, the region composed of a base sequence that promotes localization of the guide RNA to the nucleus is a region composed of a base sequence derived from a small RNA. In some embodiments, the region composed of a base sequence that promotes localization of the guide RNA to the nucleus is a region composed of an snRNA sequence.

In some embodiments, the region composed of a base sequence that promotes localization of the guide RNA to the cytoplasm is a region composed of a base sequence derived from a small RNA. In some embodiments, the region composed of a base sequence that promotes localization of the guide RNA to the cytoplasm is a region composed of a base sequence that promotes localization of the guide RNA to a ribosome, and in some embodiments, a region composed of an rRNA sequence. In some embodiments, the region composed of a base sequence that promotes localization of the guide RNA to the cytoplasm is a region composed of a transfer RNA (tRNA) sequence. In some embodiments, the region composed of a base sequence that promotes localization of the guide RNA to the cytoplasm is a region composed of a 7SL RNA sequence that is an RNA sequence derived from a signal recognition particle (SRP).

In some embodiments, the region composed of a base sequence that promotes double strand formation between the target RNA and the antisense region is a region composed of a base sequence that thermodynamically and conformationally promotes formation of a double strand and a complex containing a double strand (Nat. Biotechnol., 2019, Vol. 37, pp. 657-666). In some embodiments, the region composed of a base sequence that promotes double strand formation between the target RNA and the antisense region is a region composed of an snRNA sequence, a region composed of a base sequence to form a stem-loop structure, or any combination thereof. In some embodiments, the region composed of a base sequence that promotes double strand formation between the target RNA and the antisense region is a region composed of a U6 snRNA sequence, a region composed of a U1 snRNA sequence, a region composed of a U7 snRNA sequence, or a region composed of a base sequence to form a stem-loop structure, or any combination thereof.

In some embodiments, the region composed of a base sequence that inhibits non-specific double strand formation by the antisense region is a region composed of a base sequence that thermodynamically and conformationally reduces the off-target effect. In some embodiments, the region composed of a base sequence that inhibits non-specific double strand formation by the antisense region is a region composed of a base sequence to form a stem-loop structure (Chem. Commun., 2018, Vol. 54, pp. 2377-2380).

In some embodiments, the region composed of a base sequence that promotes stabilization of a complex formed of the target RNA and the antisense region is a region composed of a base sequence that shows resistance to nuclease activity by thermodynamically and conformationally stabilizing a double strand and a complex containing a double strand (Antival Chemistry & Chemotherapy, 1996, Vol. 7, pp. 86-93). In some embodiments, the region composed of a base sequence that promotes stabilization of a complex formed of the target RNA and the antisense region is a region composed of a base sequence to form a stem-loop structure.

In some embodiments, the guide RNA of the present invention contains, as the functional region, a region composed of an snRNA sequence, a region composed of an rRNA sequence, a region composed of a base sequence to form a guanine quadruplex (Gq: G-quadruplex) structure, and a region composed of a base sequence to form a stem-loop structure, a region composed of an SRP-derived RNA sequence, a region composed of a long non-coding RNA (IncRNA) sequence, a region composed of a tRNA sequence, a region composed of a small nucleolar RNA (snoRNA) sequence, or a region composed of an miRNA sequence, or any combination thereof. In some embodiments, the guide RNA of the present invention contains, as the functional region, a region composed of an snRNA sequence, a region composed of an rRNA sequence, a region composed of a base sequence to form a Gq structure, or a region composed of a base sequence to form a stem-loop structure, or any combination thereof.

In some embodiments, the guide RNA of the present invention contains, as the functional region, a region composed of an snRNA sequence. In some embodiments, the guide RNA of the present invention contains, as the functional region, a region composed of an snRNA sequence and a region composed of a base sequence to form a stem-loop structure. The region, which is composed of a base sequence to form a stem-loop structure, and can be used in combination with the region composed of an snRNA sequence, is, in some embodiments, a region composed of an artificial stem-loop sequence. The artificial stem-loop sequence is a sequence to form an artificially produced stem-loop structure, and, for example, an artificial stem-loop sequence (referred to as "STL sequence" in the present description) contained in a U6 cassette or a 5S cassette (Gene Ther., 1997, Vol. 4, pp. 45-54, Nat. Biotechnol., 2002, Vol. 20, pp. 505-508, Mol. Ther., 2003, Vol. 7, pp. 237-247) is exemplified. The region, which is composed of a base sequence to form a stem-loop structure, and can be used in combination with the region composed of an snRNA sequence, is, in some embodiments, a region composed of an STL sequence. In some embodiments, the guide RNA of the present invention contains, as the functional region, a region composed of an snRNA sequence and a region composed of an artificial stem-loop sequence. In some embodiments, the guide RNA of the present invention contains, as the functional region, a region composed of an snRNA sequence and a region composed of an STL sequence.

The region composed of an snRNA sequence used in the present invention is a region composed of the base sequence of an snRNA well known to those skilled in the art, and may partially contain a modification in the base sequence of a natural snRNA as long as it has the function of the snRNA sequence, and/or may be a partial sequence of the base sequence of a natural snRNA as long as it has the function of the snRNA sequence. For example, the guide RNA of the present invention contains, as the functional region, a region composed of a U1 snRNA sequence, a U2 snRNA sequence, a U4 snRNA sequence, a U5 snRNA sequence, a U6 snRNA sequence, a U7 snRNA sequence, a U11 snRNA sequence, a U12 snRNA sequence, a U4atac snRNA sequence, or a U6atac snRNA sequence, or any combination thereof. In some embodiments, the guide RNA of the present invention contains, as the functional region, a region composed of a U1 snRNA sequence, a U2 snRNA sequence, a U4 snRNA sequence, a U5 snRNA sequence, a U6 snRNA sequence, or a U7 snRNA sequence, or any combination thereof. In some embodiments, the guide RNA of the present invention contains, as the functional region, a region composed of a U6 snRNA sequence, a region composed of a U1 snRNA sequence, or a region composed of a U7 snRNA sequence.

In some embodiments, the guide RNA of the present invention contains, as the functional region, a region composed of a U6 snRNA sequence, a region composed of a U1 snRNA sequence, or a region composed of a U7 snRNA sequence, and a region composed of a base sequence to form a stem-loop structure. In some embodiments, the guide RNA of the present invention contains, as the functional region, a region composed of a U6 snRNA sequence and a region composed of a base sequence to form a stem-loop structure. In some embodiments, the guide RNA of the present invention contains, as the functional region, a region composed of a U6 snRNA sequence and a region composed of an artificial stem-loop sequence. In some embodiments, the guide RNA of the present invention contains, as the functional region, a region composed of a U6 snRNA sequence and a region composed of an STL sequence. In some embodiments, the guide RNA of the present invention contains, as the functional region, a region composed of a U1 snRNA sequence and a region composed of a base sequence to form a stem-loop structure. In some embodiments, the guide RNA of the present invention contains, as the functional region, a region composed of a U1 snRNA sequence and a region composed of an artificial stem-loop sequence. In some embodiments, the guide RNA of the present invention contains, as the functional region, a region composed of a U1 snRNA sequence and a region composed of an STL sequence. In some embodiments, the guide RNA of the present invention contains, as the functional region, a region composed of a U7 snRNA sequence and a region composed of a base sequence to form a stem-loop structure. In some embodiments, the guide RNA of the present invention contains, as the functional region, a region composed of a U7 snRNA sequence and a region composed of an artificial stem-loop sequence. In some embodiments, the guide RNA of the present invention contains, as the functional region, a region composed of a U7 snRNA sequence and a region composed of an STL sequence.

The region composed of a U6 snRNA sequence used in the present invention is a region composed of the base sequence of U6 snRNA, and may partially contain a modification in the base sequence of natural U6 snRNA as long as it has the function of the snRNA sequence, and/or may be a partial sequence of natural U6 snRNA as long as it has the function of the snRNA sequence. In some embodiments, the region composed of a U6 snRNA sequence used in the present invention is a region composed of a partial sequence of the base sequence of U6 snRNA, and in some embodiments, a region composed of a base sequence from the transcription start site to position 27 of the base sequence of U6 snRNA. In the guide RNA of the present invention, the region composed of a U6 snRNA sequence may be used in combination with a region composed of an artificial stem-loop sequence as long as it has the function of the snRNA sequence. When the region composed of a U6 snRNA sequence and a region composed of an artificial stem-loop sequence are used, the antisense region is inserted between the region composed of a U6 snRNA sequence and the region composed of an artificial stem-loop sequence. In some embodiments, the region composed of a U6 snRNA sequence used in the present invention is a region composed of the base sequence represented by SEQ ID NO: 1 or a base sequence, in which 1 to 3 bases have been deleted, substituted, inserted, and/or added in the base sequence represented by SEQ ID NO: 1, and which has the function of the snRNA sequence. In some embodiments, the region composed of an artificial stem-loop sequence used in the present invention is a region composed of an STL sequence, and in some embodiments, a region composed of the base sequence represented by SEQ ID NO: 2 or a base sequence, in which 1 to 3 bases have been deleted, substituted, inserted, and/or added in the base sequence represented by SEQ ID NO: 2, and which forms a stem-loop structure.

The region composed of a U1 snRNA sequence used in the present invention is a region composed of the base sequence of U1 snRNA, and may partially contain a modification in the base sequence of natural U1 snRNA as long as it has the function of the snRNA sequence, and/or may be a partial sequence of natural U1 snRNA as long as it has the function of the snRNA sequence. In the guide RNA of the present invention, the region composed of a U1 snRNA sequence may be used by linking the antisense region to the U1 snRNA sequence, or may be used by inserting the antisense region into the U1 snRNA sequence as long as it has the function of the snRNA sequence. When the antisense region is inserted into the U1 snRNA sequence, the U1 snRNA sequence is divided into any two regions, and the antisense region is inserted therebetween. The insertion of the antisense region into the U1 snRNA sequence may be carried out by substituting a portion of the U1 snRNA sequence with the antisense region. In some embodiments, a base sequence from position 3 to position 10 from the transcription start site of the base sequence of the U1 snRNA sequence can be substituted with the antisense region. In the insertion of the antisense region into the U1 snRNA sequence, in some embodiments, the antisense region can be inserted at position 3 from the transcription start site of the U1 snRNA sequence composed of a base sequence in which 8 bases from position 3 to position 10 from the transcription start site of the base sequence of the U1 snRNA sequence have been deleted. In some embodiments, the region composed of a U1 snRNA sequence used in the present invention is a region composed of the base sequence represented by SEQ ID NO: 3 or a base sequence, in which 1 to 20 bases have been deleted, substituted, inserted, and/or added in the base sequence represented by SEQ ID NO: 3, and which has the function of the snRNA sequence. In some embodiments, it is a region composed of a base sequence, in which 8 bases from position 3 to position 10 from the transcription start site have been deleted in the base sequence represented by SEQ ID NO: 3, and which has the function of the snRNA sequence.

The region composed of a U7 snRNA sequence used in the present invention is a region composed of the base sequence of U7 snRNA, and may partially contain a modification in the base sequence of natural snRNA as long as it has the function of the snRNA sequence, and/or may be a partial sequence of natural U7 snRNA as long as it has the function of the snRNA sequence. In some embodiments, the region composed of a U7 snRNA sequence used in the present invention is a region composed of a partial sequence of the base sequence of U7 snRNA. In some embodiments, the region composed of a U7 snRNA sequence used in the present invention is a region composed of a partial sequence of the base sequence of U7 snRNA, and in some embodiments, a region composed of a U7 snRNA sequence containing an Sm protein binding site (Sm OPT sequence) and the stem-loop sequence of U7 snRNA. In the guide RNA of the present invention, the region composed of a U7 snRNA sequence may be used by linking the antisense region to the U7 snRNA sequence, or may be used by inserting the antisense region into the U7 snRNA sequence as long as it has the function of the snRNA sequence. The region composed of a U7 snRNA sequence used in the present invention is a region composed of the base sequence represented by SEQ ID NO: 4 or a base sequence, in which 1 to 10 bases have been deleted, substituted, inserted, and/or added in the base sequence represented by SEQ ID NO: 4, and which has the function of the snRNA sequence.

In some embodiments, the guide RNA of the present invention contains, as the functional region, a region composed of an rRNA sequence. In some embodiments, the guide RNA of the present invention contains, as the functional region, a region composed of an rRNA sequence and a region composed of a base sequence to form a stem-loop structure.

The region composed of an rRNA sequence used in the present invention is a region composed of the base sequence of an rRNA well known to those skilled in the art, and may partially contain a modification in the base sequence of natural rRNA as long as it has the function of the rRNA sequence. In some embodiments, the guide RNA of the present invention contains, as the functional region, a region composed of a eukaryotic rRNA sequence. In some embodiments, the guide RNA of the present invention contains, as the functional region, a region composed of a 28S rRNA sequence, a 18S rRNA sequence, a 5.8S rRNA sequence, a 5S rRNA sequence, a mitochondrial 12S rRNA sequence, or a mitochondrial 16S rRNA sequence, or any combination thereof. In some embodiments, the guide RNA of the present invention contains, as the functional region, a region composed of a 5S rRNA sequence.

The region composed of a 5S rRNA sequence used in the present invention is a region composed of the base sequence of 5S rRNA, and may partially contain a modification in the base sequence of natural 5S rRNA as long as it has the function of the rRNA sequence. In the guide RNA of the present invention, the region composed of a 5S rRNA sequence may be used in combination with a region composed of an artificial stem-loop sequence as long as it has the function of the rRNA sequence. When the region composed of a 5S rRNA sequence and a region composed of an artificial stem-loop sequence are used, the antisense region is inserted between the region composed of a 5S rRNA sequence and the region composed of an artificial stem-loop sequence. In some embodiments, the region composed of a 5S rRNA sequence used in the present invention is a region composed of the base sequence represented by SEQ ID NO: 5 or a base sequence, in which 1 to 10 bases have been deleted, substituted, inserted, and/or added in the base sequence represented by SEQ ID NO: 5, and which has the function of the rRNA sequence. In some embodiments, the region composed of an artificial stem-loop sequence used in the present invention is a region composed of the base sequence represented by SEQ ID NO: 2 or a base sequence, in which 1 to 3 bases have been deleted, substituted, inserted, and/or added in the base sequence represented by SEQ ID NO: 2, and which forms a stem-loop structure.

In some embodiments, the guide RNA of the present invention contains, as the functional region, a region composed of a base sequence to form a guanine quadruplex structure, a region composed of a base sequence to form a stem-loop structure, or a combination thereof. In some embodiments, the guide RNA of the present invention contains, as the functional region, a region composed of a base sequence to form a guanine quadruplex structure. In some embodiments, the guide RNA of the present invention contains, as the functional region, a region composed of a base sequence to form a stem-loop structure.

The region composed of a base sequence to form a guanine quadruplex structure (Gq structure) used in the present invention contains a Gq sequence. The Gq sequence is a sequence containing four repeating units composed of consecutive guanine bases, and four guanine bases form a quadruplex (Gq) structure on a plane. Abase other than guanine may be contained between the repeating units composed of guanine as long as the Gq structure is maintained. In some embodiments, the Gq sequence contains 1, 2, 3, or 4 repeating units composed of consecutive guanine bases, and forms 1-, 2-, 3-, or 4-layer Gq, respectively. In some embodiments, the Gq sequence contains 3 repeating units composed of consecutive guanine bases, and forms 3-layer Gq. This is referred to as a "3-layer guanine quadruplex structure" (hereinafter, also referred to as "3Gq"). In some embodiments, the guide RNA of the present invention contains, as the functional region, a region composed of a base sequence to form 3Gq, and in some embodiments, the region composed of a base sequence to form 3Gq is a region composed of the base sequence represented by SEQ ID NO: 6 or a base sequence, in which 1 to 3 bases have been deleted, substituted, inserted, and/or added in the base sequence represented by SEQ ID NO: 6, and which forms a Gq structure.

The stem-loop structure is also referred to as a hairpin structure and is well known in the technical field. Examples of the base sequence to form a stem-loop structure include an aptamer sequence (Int. J. Biochem. Mol. Biol., 2013, Vol. 4, pp. 27-40, WO 2016/143700), a BoxB sequence-derived sequence, an MS2 sequence-derived sequence, a PP7 sequence-derived sequence (Integr. Biol., 2009, Vol. 1, pp. 499-505, Nuclear Acids Research, 2016, Vol. 44, pp. 9555-9564), an artificial stem-loop sequence (for example, an artificial stem-loop sequence (STL sequence) contained in a U6 cassette or a 5S cassette) (Gene Ther., 1997, Vol. 4, pp.45-54, Nat. Biotechnol., 2002, Vol. 20, pp. 505-508, Mol. Ther., 2003, Vol. 7, pp. 237-247)), and the like.

In some embodiments, the guide RNA of the present invention contains, as the functional region, a region composed of a base sequence to form a stem-loop structure, and the region composed of a base sequence to form a stem-loop structure is a BoxB sequence-derived sequence, an aptamer sequence, an MS2 sequence-derived sequence, a PP7 sequence-derived sequence, a region composed of an artificial stem-loop sequence (for example, an STL sequence), or any combination thereof. In some embodiments, the guide RNA of the present invention contains, as the functional region, a region composed of a BoxB sequence-derived sequence. In some embodiments, the guide RNA of the present invention contains, as the functional region, a region composed of an STL sequence.

In some embodiments, the region composed of a BoxB sequence-derived sequence used in the present invention is composed of the base sequence represented by SEQ ID NO: 7 or a base sequence, in which 1 to 3 bases have been deleted, substituted, inserted, and/or added in the base sequence represented by SEQ ID NO: 7, and which forms a stem-loop structure.

In some embodiments, the region composed of an artificial stem-loop sequence used in the present invention is a region composed of an artificial stem-loop sequence (STL sequence) contained in a U6 cassette or a 5S cassette, and in some embodiments, a region composed of the base sequence represented by SEQ ID NO: 2 or a base sequence, in which 1 to 3 bases have been deleted, substituted, inserted, and/or added in the base sequence represented by SEQ ID NO: 2, and which forms a stem-loop structure.

In some embodiments, the guide RNA of the present invention contains, as the functional region, a region composed of an SRP-derived RNA sequence, and in some embodiments, the region composed of an SRP-derived RNA sequence is a region composed of a 7SL RNA sequence, a 6S RNA sequence, or 4.5S RNA sequence.

In some embodiments, the guide RNA of the present invention may contain at least two identical functional regions, and in other embodiments, at least one each of two or more different functional regions.

### 4. Linkage of Functional Regions

In the guide RNA of the present invention, the functional region is linked to the antisense region. The "linkage" as used herein includes direct binding and binding via a linker. In some embodiments, in the guide RNA of the present invention, at least one functional region is directly bound to the antisense region. In other embodiments, in the guide RNA of the present invention, at least one functional region is linked to the antisense region via a linker. When a plurality of functional regions are linked and used in the guide RNA of the present invention, the linkage between the functional regions may be either direct binding or linkage via a linker.

When a linker is used, in some embodiments, the length of the linker is 1 to 10 bases, in some embodiments, 1 to 6 bases, in some embodiments, 1 to 3 bases, in some embodiments, 3 to 6 bases, in some embodiments, 3 bases, and in some embodiments, 6 bases. The base sequence of the linker can be appropriately designed by those skilled in the art based on the sequence constituting the guide RNA. For example, the base sequence of the linker used in the present invention may be designed so as not to form a complementary strand with the target RNA. In some embodiments, the linker is composed of a base sequence "UCU", "GUCGAC" which is a restriction enzyme SalI site sequence, or "UCUAGA" which is a restriction enzyme XbaI site sequence.

In some embodiments, when the guide RNA of the present invention contains, as the functional region, a region composed of a BoxB sequence-derived sequence, linkage via a linker can be used between the region composed of a BoxB sequence-derived sequence and the antisense region and between the region composed of a BoxB sequence-derived sequence and another functional region, and in some embodiments, the linker is a base sequence "UCU". In some embodiments, when the guide RNA of the present invention contains, as the functional region, a region composed of a U6 snRNA sequence and an optionally contained region composed of a base sequence to form a stem-loop structure, or when the guide RNA of the present invention contains, as the functional region, a region composed of a 5S rRNA sequence and an optionally contained region composed of a base sequence to form a stem-loop structure, the antisense region can be linked to the functional region via a linker, and in some cases, the linker is a base sequence "GUCGAC" or "UCUAGA".

### 5. Guide RNA for Editing Target RNA

The guide RNA of the present invention is a guide RNA that does not substantially contain an ADAR-recruiting base sequence. The "ADAR-recruiting base sequence" is a base sequence in which a stem-loop structure is formed in the same molecule, and ADAR binds to the stem-loop structure so that the ADAR can be recruited to a target RNA. As an example of the ADAR-recruiting base sequence, a base sequence designed based on a stem-loop structure derived from an ADAR substrate such as an mRNA precursor of GluR2 (WO 2016/097212, WO 2017/050306, German Patent No. 102015012522, WO 2017/010556, WO 2019/111957, Nucleic Acids Research, 2017, Vol. 45, pp. 2797-2808, Nature Methods, 2019, Vol. 16, pp. 239-242) is exemplified. In the present invention, the phrase "does not substantially contain" an ADAR-recruiting base sequence means that an ADAR-recruiting base sequence is not contained in the same molecule of the guide RNA.

In some embodiments, the guide RNA of the present invention is either of the following guide RNAs:
a guide RNA which contains at least one functional region and an antisense region that is complementary to a portion of the target RNA and forms a double strand with the target RNA, wherein the at least one functional region is directly bound to the antisense region, and wherein the guide RNA does not substantially contain an ADAR-recruiting base sequence, or
a guide RNA which contains at least one functional region and an antisense region that is complementary to a portion of the target RNA and forms a double strand with the target RNA, wherein the at least one functional region is linked to the antisense region via a linker, and wherein the guide RNA does not substantially contain an ADAR-recruiting base sequence.

In some embodiments, the guide RNA of the present invention is the following guide RNA:
a guide RNA which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein the antisense region has a base to form a mismatched base pair with the target RNA.

In some embodiments, the guide RNA of the present invention is a guide RNA which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence, and the guide RNA has the following characteristic:
(1) the functional region is a region composed of a base sequence having any one or more functions among functions including stabilization of the guide RNA, localization of the guide RNA to the nucleus, localization of the guide RNA to the cytoplasm, promotion of double strand formation between the target RNA and the antisense region, inhibition of non-specific double strand formation by the antisense region, and stabilization of a complex formed of the target RNA and the antisense region,
(2) the functional region is a region composed of an snRNA sequence, a region composed of an rRNA sequence, a region composed of a base sequence to form a guanine quadruplex structure, or a region composed of a base sequence to form a stem-loop structure, or any combination thereof,
(3) the functional region is a region composed of an snRNA sequence and an optionally contained region composed of a base sequence to form a stem-loop structure,
(4) the functional region is a region composed of an snRNA sequence selected from a region composed of a U6 snRNA sequence, a region composed of a U1 snRNA sequence, and a region composed of a U7 snRNA sequence, and an optionally contained region composed of a base sequence to form a stem-loop structure,
(5) the functional region is a region composed of a U6 snRNA sequence and a region composed of an artificial stem-loop sequence,
(6) the functional region is a region composed of a base sequence, which is a partial sequence of the base sequence of a U6 snRNA sequence and has the function of the snRNA sequence, and a region composed of an artificial stem-loop sequence,
(7) the functional region is a region composed of a base sequence, which is a partial sequence from the transcription start site to position 27 of the base sequence of a U6 snRNA sequence and has the function of the snRNA sequence, and a region composed of an artificial stem-loop sequence,
(8) the functional region is a region composed of the base sequence represented by SEQ ID NO: 1 or a base sequence, in which 1 to 3 bases have been deleted, substituted, inserted, and/or added in the base sequence represented by SEQ ID NO: 1, and which has the function of the snRNA sequence, and a region composed of the base sequence represented by SEQ ID NO: 2 or a base sequence, in which 1 to 3 bases have been deleted, substituted, inserted, and/or added in the base sequence represented by SEQ ID NO: 2, and which forms a stem-loop structure,
(9) the functional region is a region composed of a U1 snRNA sequence,
(10) the functional region is a region composed of the base sequence represented by SEQ ID NO: 3 or a base sequence, in which 1 to 20 bases have been deleted, substituted, inserted, and/or added in the base sequence represented by SEQ ID NO: 3, and which has the function of the snRNA sequence,
(11) the functional region is a region composed of a base sequence, in which 8 bases from position 3 to position 10 from the transcription start site have been deleted in the base sequence represented by SEQ ID NO: 3, and which has the function of the snRNA sequence,
(12) the functional region is a region composed of a U7 snRNA sequence,
(13) the functional region is a region composed of a base sequence, which is a partial sequence of the base sequence of U7 snRNA containing an Sm protein binding site (Sm OPT sequence) and the stem-loop sequence of U7 snRNA, and has the function of the snRNA sequence,
(14) the functional region is a region composed of the base sequence represented by SEQ ID NO: 4 or a base sequence, in which 1 to 10 bases have been deleted, substituted, inserted, and/or added in the base sequence represented by SEQ ID NO: 4, and which has the function of the snRNA sequence,
(15) the functional region is a region composed of an rRNA sequence and an optionally contained region composed of a base sequence to form a stem-loop structure,
(16) the functional region is a region composed of a 5S rRNA sequence and an optionally contained region composed of a base sequence to form a stem-loop structure,
(17) the functional region is a region composed of the base sequence represented by SEQ ID NO: 5 or a base sequence, in which 1 to 10 bases have been deleted, substituted, inserted, and/or added in the base sequence represented by SEQ ID NO: 5, and which has the function of an rRNA sequence, and an optionally contained region composed of a base sequence to form a stem-loop structure,
(18) the functional region is a region composed of a 5S rRNA sequence and a region composed of an artificial stem-loop sequence,
(19) the functional region is a region composed of the base sequence represented by SEQ ID NO: 5 or a base sequence, in which 1 to 3 bases have been deleted, substituted, inserted, and/or added in the base sequence represented by SEQ ID NO: 5, and which has the function of an rRNA sequence, and a region composed of the base sequence represented by SEQ ID NO: 2 or a base sequence, in which 1 to 3 bases have been deleted, substituted, inserted, and/or added in the base sequence represented by SEQ ID NO: 2, and which forms a stem-loop structure,
(20) the functional region is a region composed of a base sequence to form a guanine quadruplex structure or a region composed of a base sequence to form a stem-loop structure, or a combination thereof,
(21) the functional region is a region composed of a base sequence to form a guanine quadruplex structure,
(22) the functional region is a region composed of a base sequence to form a 3-layer guanine quadruplex structure,
(23) the functional region is a region composed of the base sequence represented by SEQ ID NO: 6 or a base sequence, in which 1 to 3 bases have been deleted, substituted, inserted, and/or added in the base sequence represented by SEQ ID NO: 6, and which forms a guanine quadruplex structure,
(24) the functional region is a region composed of a base sequence to form a stem-loop structure,
(25) the functional region is a region composed of a BoxB sequence-derived sequence,
(26) the functional region is a region composed of the base sequence represented by SEQ ID NO: 7 or a base sequence, in which 1 to 3 bases have been deleted, substituted, inserted, and/or added in the base sequence represented by SEQ ID NO: 7, and which forms a stem-loop structure,
(27) the functional region is a combination of a region composed of a base sequence to form a guanine quadruplex structure and a region composed of a base sequence to form a stem-loop structure, or
(28) the functional region is a combination of a region composed of a base sequence to form a 3-layer guanine quadruplex structure and a region composed of a BoxB sequence-derived sequence.

In the guide RNA of the present invention having any of the above-mentioned characteristics (1) to (28), the at least one functional region may be directly bound to the antisense region or linked thereto via a linker.

In some embodiments, the guide RNA of the present invention is a guide RNA which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and the guide RNA has the following characteristic:
(a-1) the guide RNA is composed of a functional region composed of a U6 snRNA sequence, an antisense region, an optionally contained linker, and a region composed of an artificial stem-loop sequence, and the functional region composed of a U6 snRNA sequence, the antisense region, and the region composed of an artificial stem-loop sequence are linked in this order from the 5' side to the 3' side,
(a-2) the guide RNA is composed of a functional region composed of a base sequence, which is a partial sequence from the transcription start site to position 27 of the base sequence of a U6 snRNA sequence and has the function of the snRNA sequence, an antisense region, an optionally contained linker, and a region composed of an artificial stem-loop sequence, and the functional region composed of a base sequence, which is a partial sequence from the transcription start site to position 27 of the base sequence of a U6 snRNA sequence and has the function of the snRNA sequence, the antisense region, and the region composed of an artificial stem-loop sequence are linked in this order from the 5' side to the 3' side,
(a-3) the guide RNA is composed of a functional region composed of a U1 snRNA sequence and an antisense region, and the antisense region is inserted into the base sequence of the U1 snRNA sequence,
(a-4) the guide RNA is composed of a functional region composed of a base sequence in which 8 bases from position 3 to position 10 from the transcription start site of the base sequence of a U1 snRNA sequence have been deleted, and which has the function of the snRNA sequence, and an antisense region, and the antisense region is inserted at position 3 from the transcription start site of the base sequence of the U1 snRNA sequence,
(a-5) the guide RNA is composed of a functional region composed of a U7 snRNA sequence and an antisense region, and the antisense region and the functional region composed of a U7 snRNA sequence are linked in this order from the 5' side to the 3' side,
(a-6) the guide RNA is composed of a functional region composed of a base sequence, which is a partial sequence of the base sequence of a U7 snRNA sequence containing an Sm protein binding site (Sm OPT sequence) and the stem-loop sequence of U7 snRNA, and has the function of the snRNA sequence, and an antisense region, and the antisense region and the functional region composed of a partial sequence of the base sequence of the U7 snRNA sequence are linked in this order from the 5' side to the 3' side,
(a-7) the guide RNA is composed of a functional region composed of a 5S rRNA sequence, an antisense region, an optionally contained linker, and a region composed of an artificial stem-loop sequence, and the functional region composed of a 5S rRNA sequence, the antisense region, and the region composed of an artificial stem-loop sequence are linked in this order from the 5' side to the 3' side,
(a-8) the guide RNA is composed of an antisense region, a functional region composed of a base sequence to form a guanine quadruplex structure, and an optionally contained linker, and the antisense region and the functional region composed of a base sequence to form a guanine quadruplex structure are linked in this order from the 5' side to the 3' side,
(a-9) the guide RNA is composed of a functional region composed of a base sequence to form a guanine quadruplex structure, an antisense region, and an optionally contained linker, and the functional region composed of a base sequence to form a guanine quadruplex structure and the antisense region are linked in this order from the 5' side to the 3' side,
(a-10) the guide RNA is composed of an antisense region, a functional region composed of a base sequence to form a guanine quadruplex structure, an additional functional region, and an optionally contained linker, and the antisense region, the functional region composed of a base sequence to form a guanine quadruplex structure, and the additional functional region are linked in this order from the 5' side to the 3' side,
(a-11) the guide RNA is composed of an antisense region, a functional region composed of a base sequence to form a guanine quadruplex structure, an additional functional region, and an optionally contained linker, and the additional functional region, the antisense region, and the functional region composed of a base sequence to form a guanine quadruplex structure are linked in this order from the 5' side to the 3' side,
(a-12) the guide RNA is composed of an antisense region, a functional region composed of a base sequence to form a stem-loop structure, and an optionally contained linker, and the antisense region and the functional region composed of a base sequence to form a stem-loop structure are linked in this order from the 5' side to the 3' side,
(a-13) the guide RNA is composed of a functional region composed of a base sequence to form a stem-loop structure, an antisense region, and an optionally contained linker, and the functional region composed of a base sequence to form a stem-loop structure and the antisense region are linked in this order from the 5' side to the 3' side,
(a-14) the guide RNA is composed of a functional region composed of a base sequence to form a stem-loop structure, an antisense region, an additional functional region, and an optionally contained linker, and the functional region composed of a base sequence to form a stem-loop structure, the antisense region, and the additional functional region are linked in this order from the 5' side to the 3' side,
(a-15) the guide RNA is composed of a functional region composed of a base sequence to form a guanine quadruplex structure, an antisense region, a functional region composed of a base sequence to form a stem-loop structure, and an optionally contained linker, and the functional region composed of a base sequence to form a guanine quadruplex structure, the antisense region, and the functional region composed of a base sequence to form a stem-loop structure are linked in this order from the 5' side to the 3' side,
(a-16) the guide RNA is composed of a functional region composed of a base sequence to form a stem-loop structure, an antisense region, a functional region composed of a base sequence to form a guanine quadruplex structure, and an optionally contained linker, and the functional region composed of a base sequence to form a stem-loop structure, the antisense region, and the functional region composed of a base sequence to form a guanine quadruplex structure are linked in this order from the 5' side to the 3' side,
(a-17) the guide RNA is composed of an antisense region, a functional region composed of a base sequence to form a guanine quadruplex structure, a functional region composed of a base sequence to form a stem-loop structure, and an optionally contained linker, and the antisense region, the functional region composed of a base sequence to form a guanine quadruplex structure, and the functional region composed of a base sequence to form a stem-loop structure are linked in this order from the 5' side to the 3' side, or
(a-18) the guide RNA is composed of a functional region composed of a base sequence to form a guanine quadruplex structure, a functional region composed of a base sequence to form a stem-loop structure, an antisense region, and an optionally contained linker, and the functional region composed of a base sequence to form a guanine quadruplex structure, the functional region composed of a base sequence to form a stem-loop structure, and the antisense region are linked in this order from the 5' side to the 3' side,

### <ADAR Used in Present Invention>

The ADAR used in the present invention also includes naturally occurring ADAR, and variants thereof as long as they have deaminase activity. The deaminase activity can be measured by detecting deamination of a substrate by a method known to those skilled in the art. For example, the deaminase activity can be measured by detecting conversion of adenosine to inosine or conversion of cytidine to uridine. Specifically, the deaminase activity can be measured by the method described in Example 7 or 8. The ADAR used in the present invention is, in some embodiments, eukaryote-derived ADAR, in some embodiments, mammal-derived ADAR, and in some embodiments, human ADAR. The ADAR used in the present invention is, in some embodiments, ADAR1 or ADAR2, and in some embodiments, ADAR2. ADAR1 includes two splicing variants ADAR1 p110 and ADAR1 p150, and the ADAR used in the present invention is, in some embodiments, ADAR1 p110 or ADAR1 p150. The ADAR used in the present invention is, in some embodiments, human ADAR1 or human ADAR2, and in some embodiments, human ADAR2 The ADAR used in the present invention is, in some embodiments, a polypeptide containing a double-stranded RNA binding domain (dsRBD) and having deaminase enzyme activity (Trends in Biochemical Sciences, 2001, Vol. 26, pp. 376-384, RNA, 2001, Vol. 7, pp. 846-858). The ADAR used in the present invention is, in some embodiments, a polypeptide that contains a deaminase domain and can convert adenosine of the target RNA to inosine. The ADAR used in the present invention is, in other embodiments, a polypeptide that contains a deaminase domain and can convert cytidine of the target RNA to uridine. The ADAR used in the present invention is, in still other embodiments, a polypeptide that contains a deaminase domain and can convert adenosine of the target RNA to inosine and cytidine thereof to uridine. The ADAR used in the present invention may be a fusion protein with another factor. The variant of ADAR used in the present invention includes a variant having adenosine deaminase activity, a variant having cytidine deaminase activity, and a variant that recognizes both adenosine and cytidine and has deaminase activity to convert adenosine and cytidine to inosine and uridine, respectively (Science, 2019, Vol. 365, pp. 382-386). The ADAR and variants of ADAR used in the present invention may be fusion proteins with another factor.

In some embodiments, the ADAR used in the present invention is a polypeptide composed of the amino acid sequence set forth in Accession No. [NP_001103.1], Accession No. [NP_056648.1], or Accession No. [NP_001102.3], or a polypeptide that is composed of an amino acid sequence having 90% or more identity to any of these amino acid sequences or an amino acid sequence, in which 1 to 10 amino acids have been deleted, substituted, inserted, and/or added in any of these amino acid sequences, and that has adenosine deaminase activity. The ADAR used in the present invention is, in some embodiments, a polypeptide composed of the amino acid sequence represented by SEQ ID NO: 9 (human ADAR2) or a polypeptide that is composed of an amino acid sequence having 90% or more identity to the sequence or an amino acid sequence, in which 1 to 10 amino acids have been deleted, substituted, inserted, and/or added in the sequence, and that has adenosine deaminase activity. The ADAR used in the present invention is, in some embodiments, ADAR endogenously present in a eukaryotic cell, and in some embodiments, may be ADAR exogenously introduced into a eukaryotic cell. The introduction of ADAR into a eukaryotic cell may be carried out by directly introducing an ADAR polypeptide, or introducing an expression vector containing a nucleic acid encoding ADAR.

The "identity" as used herein means a value (Identity) obtained using parameters provided as the default by searching using the EMBOSS NEEDLE program (J. Mol. Biol., 1970, Vol. 48, pp. 443-453). The parameters are as follows.
Gap Open penalty = 10
Gap Extend penalty = 0.5
Matrix = EBLOSUM62

### <System for Editing Target RNA of Present Invention>

The present invention also provides a system for editing a target RNA including the guide RNA of the present invention and ADAR. The system for editing a target RNA sequence including the guide RNA of the present invention and ADAR includes a kit for editing a target RNA sequence including the guide RNA of the present invention and ADAR, or a method for editing a target RNA sequence using the guide RNA of the present invention and ADAR. In some embodiments, the system for editing a target RNA of the present invention is a system including a guide RNA which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence, and the system including ADAR, and in some embodiments, a system including a guide RNA which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence, and the system including ADAR1 or ADAR2

The system for editing a target RNA of the present invention can be used both intracellularly and extracellularly. In some embodiments, the system can be used in a eukaryotic cell. In some embodiments, the system can be used in a prokaryotic cell, in a bacterium, in a phage, in a virus, or the like.

### <Nucleic Acid Encoding Guide RNA of Present Invention>

The present invention also provides a nucleic acid encoding the guide RNA of the present invention (also referred to as "the nucleic acid of the present invention" in this section). The nucleic acid of the present invention is a nucleic acid encoding a guide RNA which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence. In some embodiments, the nucleic acid of the present invention is a nucleic acid encoding a guide RNA which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein a nucleic acid encoding the functional region includes the following nucleic acids:
(1) a nucleic acid composed of a base sequence represented by one SEQ ID NO selected from SEQ ID NOS shown in the following "Nucleic Acids Encoding Functional Region",
(2) a nucleic acid composed of a base sequence, in which 1 to 20 bases have been deleted, substituted, inserted, and/or added in the base sequence represented by SEQ ID NO: 12 shown in the following "Nucleic Acids Encoding Functional Region", and which has a function as the functional region,
(3) a nucleic acid composed of a base sequence, in which 1 to 10 bases have been deleted, substituted, inserted, and/or added in the base sequence represented by SEQ ID NO: 13 or 14 shown in the following "Nucleic Acids Encoding Functional Region", and which has a function as the functional region,
(4) a nucleic acid composed of a base sequence, in which 1 to 3 bases have been deleted, substituted, inserted, and/or added in the base sequence represented by SEQ ID NO: 10, 11, 15, or 16 shown in the following "Nucleic Acids Encoding Functional Region", and which encodes a region having a function as the functional region, or
(5) some combinations of nucleic acids included in (1) to (3):
   Nucleic Acids Encoding Functional Region
   Nucleic acid encoding U6 snRNA: SEQ ID NO: 10
   Nucleic acid encoding STL: SEQ ID NO: 11
   Nucleic acid encoding U1 snRNA: SEQ ID NO: 12
   Nucleic acid encoding U7 snRNA: SEQ ID NO: 13
   Nucleic acid encoding 5S rRNA: SEQ ID NO: 14
   Nucleic acid encoding 3Gq: SEQ ID NO: 15
   Nucleic acid encoding BoxB: SEQ ID NO: 16

The nucleic acid of the present invention has a structure in which a deoxyribonucleic acid is linked, and is, for example, a DNA, and is, in some embodiments, a DNA. In some embodiments, the nucleic acid of the present invention is a nucleic acid integrated into an expression vector. In some embodiments, the nucleic acid of the present invention is a nucleic acid integrated into a plasmid vector. In some embodiments, the nucleic acid of the present invention is a nucleic acid integrated into a virus vector.

A specific embodiment of the guide RNA encoded by the nucleic acid of the present invention is the same as the embodiment of the guide RNA of the present invention described in the above section <Guide RNA of Present Invention>. Specific examples are shown below. In some embodiments, the nucleic acid of the present invention is a nucleic acid encoding any of the following guide RNAs:
a guide RNA which contains at least one functional region and an antisense region that is complementary to a portion of the target RNA and forms a double strand with the target RNA, wherein the at least one functional region is directly bound to the antisense region, and wherein the guide RNA does not substantially contain an ADAR-recruiting base sequence,
a guide RNA which contains at least one functional region and an antisense region that is complementary to a portion of the target RNA and forms a double strand with the target RNA, wherein the at least one functional region is linked to the antisense region via a linker, and wherein the guide RNA does not substantially contain an ADAR-recruiting base sequence, or
a guide RNA which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein the antisense region has a base to form a mismatched base pair with the target RNA.

In some embodiments, the nucleic acid of the present invention is a nucleic acid encoding a guide RNA, which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein the guide RNA has the following characteristic:
(1) the functional region is a region composed of a base sequence having any one or more functions among functions including stabilization of the guide RNA, localization of the guide RNA to the nucleus, localization of the guide RNA to the cytoplasm, promotion of double strand formation between the target RNA and the antisense region, inhibition of non-specific double strand formation by the antisense region, and stabilization of a complex formed of the target RNA and the antisense region,
(2) the functional region is a region composed of an snRNA sequence, a region composed of an rRNA sequence, a region composed of a base sequence to form a guanine quadruplex structure, or a region composed of a base sequence to form a stem-loop structure, or any combination thereof,
(3) the functional region is a region composed of an snRNA sequence and an optionally contained region composed of a base sequence to form a stem-loop structure,
(4) the functional region is a region composed of an snRNA sequence selected from a region composed of a U6 snRNA sequence, a region composed of a U1 snRNA sequence, and a region composed of a U7 snRNA sequence, and an optionally contained region composed of a base sequence to form a stem-loop structure,
(5) the functional region is a region composed of an rRNA sequence and an optionally contained region composed of a base sequence to form a stem-loop structure,
(6) the functional region is a region composed of a 5S rRNA sequence and an optionally contained region composed of a base sequence to form a stem-loop structure,
(7) the functional region is a region composed of a base sequence to form a guanine quadruplex structure or a region composed of a base sequence to form a stem-loop structure, or a combination thereof,
(8) the guide RNA is composed of an antisense region, a functional region composed of a base sequence to form a guanine quadruplex structure, an optionally contained additional functional region, and an optionally contained linker, and the antisense region, the functional region composed of a base sequence to form a guanine quadruplex structure, and the optionally contained additional functional region are linked in this order from the 5' side to the 3' side, or
(9) the guide RNA is composed of a functional region composed of a base sequence to form a stem-loop structure, an antisense region, an optionally contained additional functional region, and an optionally contained linker, and the functional region composed of a base sequence to form a stem-loop structure and the antisense region are linked in this order from the 5' side to the 3' side.

### <Nucleic Acid Encoding ADAR Used in Present Invention>

The nucleic acid encoding ADAR used in the present invention is a nucleic acid encoding ADAR described in the above <ADAR Used in Present Invention>. In some embodiments, the nucleic acid encoding ADAR used in the present invention is a nucleic acid composed of a base sequence encoding the amino acid sequence set forth in Accession No. [NP_001103.1], Accession No. [NP_056648.1], or Accession No. [NP_001102.3], or a nucleic acid that is composed of a base sequence having 90% or more identity to at least one of these sequences or a base sequence, in which 1 to 10 bases have been deleted, substituted, inserted, and/or added in at least one of these sequences, and that encodes a polypeptide having adenosine deaminase activity. In some embodiments, the nucleic acid encoding ADAR used in the present invention is a nucleic acid composed of the base sequence represented by SEQ ID NO: 8, or a nucleic acid that is composed of a base sequence having 90% or more identity to the sequence or a base sequence, in which 1 to 10 bases have been deleted, substituted, inserted, and/or added in the sequence, and that encodes a polypeptide having adenosine deaminase activity.

### <Expression Vector Containing Nucleic Acid Encoding Guide RNA of Present Invention>

The present invention also provides an expression vector containing a nucleic acid encoding the guide RNA of the present invention (also referred to as "the expression vector of the present invention"). The present invention also provides an expression vector containing a nucleic acid encoding the guide RNA of the present invention and a nucleic acid encoding ADAR. The nucleic acid encoding the guide RNA of the present invention and the nucleic acid encoding ADAR may be cloned into the same expression vector or different expression vectors. In some embodiments, the expression vector of the present invention is a combination of an expression vector containing a nucleic acid encoding the guide RNA of the present invention and an expression vector containing a nucleic acid encoding ADAR. In some embodiments, the expression vector of the present invention is an expression vector containing a nucleic acid encoding the guide RNA of the present invention and a nucleic acid encoding ADAR.

### 1. Expression Vector Used in Present Invention

The expression vector used in the present invention is not limited as long as it is an expression vector capable of expressing the guide RNA of the present invention from a nucleic acid encoding the guide RNA of the present invention and/or an expression vector capable of expressing ADAR. In some embodiments, the expression vector used in the present invention is an expression vector that can be used to express the guide RNA of the present invention and/or to express ADAR in a human cell. In some embodiments, examples of the expression vector used in the present invention include a plasmid vector, a virus vector (for example, an adenovirus vector, a retrovirus vector, and an adeno-associated virus vector), and the like.

### 2. Promoter

The expression vector of the present invention may contain a promoter operably linked to a nucleic acid encoding the guide RNA of the present invention and/or a nucleic acid encoding ADAR The phrase "operably linked" as used herein means that at least one promoter is linked to a nucleic acid so that a polypeptide or an RNA encoded by the nucleic acid can be expressed in a host cell. The promoter contained in the expression vector of the present invention is not limited, but a promoter corresponding to an RNA polymerase (for example, RNA polymerase II (polII) or RNA polymerase III (polIII)) suitable for expressing the nucleic acid encoding the guide RNA of the present invention or ADAR can be used. In order to express the guide RNA of the present invention, in some embodiments, a promoter corresponding to polII or polIII can be used, and in other embodiments, a promoter corresponding to polIII can be used. In order to express ADAR, in some embodiments, a promoter corresponding to polII can be used.

Examples of the promoter corresponding to polII include a CMV (cytomegalovirus)-derived promoter, an SV40 (simian virus 40) promoter, an RSV (respiratory syncytial virus) promoter, an EF-1α (Elongation factor 1α) promoter, a CAG promoter, a U1 snRNA promoter, a U7 snRNA promoter, and the like. Examples of the promoter corresponding to polIII include a human U6 snRNA promoter (U6) (Nat. Biotechnol., 2002, vol. 20, pp. 497-500), a high-sensitivity U6 promoter (Nucleic Acids Research, 2003, vol. 31, p. e100), a human H1 promoter, a 5S rRNA promoter, and in addition thereto, other viral and eukaryotic cell promoters known to those skilled in the art, but are not limited thereto.

The expression vector of the present invention may further contain a translation start codon, a translation stop codon, a purine base (G or A) preferable for the transcription start site of polIII, a polyA signal, a terminator sequence composed of consecutive T for polIII, an enhancer, an untranslated region, a splicing junction, or the like.

### <Host Cell of Present Invention>

The present invention also provides a host cell transformed by introducing a nucleic acid encoding the guide RNA of the present invention (also referred to as "the host cell of the present invention"). In some embodiments, the host cell of the present invention is a host cell into which the expression vector of the present invention has been introduced. In some embodiments, the host cell of the present invention is a host cell into which the expression vector of the present invention, which is a plasmid vector, has been introduced. In some embodiments, the host cell of the present invention is a host cell into which a plasmid vector for producing the expression vector of the present invention, which is a virus vector, has been introduced. In some embodiments, the host cell of the present invention is a host cell into which the expression vector of the present invention, which is a virus vector, has been introduced.

The host cell into which the expression vector of the present invention is introduced is not limited, but a cell known in the art can be selected as long as it is a cell that can be used in the production of a nucleic acid encoding the guide RNA of the present invention or the expression vector of the present invention. Examples of the host cell that can be used for replication of the vector include various cells such as natural cells commonly used in the technical field of the present invention or artificially established cells. Examples of the host cell that can be used for replication of the vector include animal cells (for example, CHO cells, HEK 293 cells, etc.), insect cells (for example, Sf9, etc.), bacteria (E. coli, etc.), yeast (genus Saccharomyces, genus Pichia, etc.), and the like). In some embodiments, E. coli can be used as the host cell. The transformation can be carried out by a method known to those skilled in the art (Green, M. R. and Sambrook, J., Molecular Cloning: A Laboratory Manual, 4th Edition, Cold Spring Harbor Laboratory Press, 2012).

### <Methods for Producing Nucleic Acid Encoding Guide RNA of Present Invention and Expression Vector of Present Invention>

The nucleic acid encoding the guide RNA of the present invention (also referred to as "the nucleic acid of the present invention" in this section) can be synthesized using a standard polynucleotide synthesis method known in the art based on the sequence described herein or publicly available sequence information. Further, if a certain nucleic acid of the present invention is obtained, by introducing a mutation into a predetermined site using a method known to those skilled in the art such as a site-directed mutagenesis method (Current Protocols in Molecular Biology edition, 1987, John Wiley & Sons Section), another nucleic acid of the present invention can also be produced.

Methods for producing the nucleic acid of the present invention and the expression vector of the present invention include a method for producing a nucleic acid including a step of culturing a host cell into which the nucleic acid of the present invention or an expression vector containing the nucleic acid of the present invention has been introduced. In some embodiments, the method for producing the nucleic acid of the present invention includes a step of culturing a host cell into which the nucleic acid of the present invention has been introduced and replicating the nucleic acid of the present invention. In some embodiments, the method of producing the nucleic acid of the present invention includes a step of culturing a host cell into which the expression vector containing the nucleic acid of the present invention has been introduced and replicating the expression vector of the present invention. When the expression vector of the present invention is a virus vector, the method for producing the expression vector of the present invention includes a step of culturing a host cell into which a virus vector plasmid containing the nucleic acid of the present invention has been introduced and purifying the virus vector produced in the host cell. The virus vector can be produced by a method known to those skilled in the art.

The methods for producing the nucleic acid of the present invention and the expression vector of the present invention may include a step of recovering a culture solution of the host cell and obtaining a lysate (bacteriolytic solution). The lysate can be obtained, for example, by treating the recovered culture solution with an alkali dissolution method or a boiling method. The method for producing the nucleic acid of the present invention may further include a step of purifying the nucleic acid or the expression vector from the lysate. Ion exchange chromatography and/or hydrophobic interaction chromatography can be used for the purification of the nucleic acid or the expression vector from the lysate. When the expression vector of the present invention is a virus vector, in the purification of the virus vector from the lysate, a cesium chloride density gradient centrifugation method, a sucrose gradient centrifugation method, an iodixanol density gradient centrifugation method, an ultrafiltration method, a diafiltration method, an affinity chromatography method, an ion exchange chromatography method, a polyethylene glycol precipitation method, an ammonium sulfate precipitation method, or the like can also be used.

### <Method for Producing Guide RNA of Present Invention>

The guide RNA of the present invention can be synthesized based on the sequence information using a standard polynucleotide synthesis method known in the art. Further, if a certain guide RNA of the present invention is obtained, by introducing a mutation into a predetermined site using a method known to those skilled in the art such as a site-directed mutagenesis method (Current Protocols in Molecular Biology edition, 1987, John Wiley & Sons Section), a variant of the guide RNA of the present invention that maintains the guiding function to the target RNA and the function of editing the target RNA can also be produced. The guide RNA of the present invention can also be produced using a modified nucleic acid. The guide RNA of the present invention can also be produced using a nucleic acid encoding the guide RNA of the present invention. For example, the guide RNA of the present invention can be produced by transcribing the guide RNA of the present invention from an expression vector containing a nucleic acid encoding the guide RNA of the present invention.

### <Polynucleotide of Present Invention>

The present invention also provides a polynucleotide for editing a target RNA by ADAR, which contains at least one functional region and an antisense region that is complementary to a portion of the target RNA and forms a double strand with the target RNA, wherein the at least one functional region is linked to the antisense region, and wherein the guide RNA does not substantially contain an ADAR-recruiting base sequence (hereinafter, also referred to as "the polynucleotide of the present invention").

The "polynucleotide" used herein includes an unmodified nucleotide (DNA or RNA) and a modified nucleotide. Examples of the modified nucleotide used in the present invention includes a nucleotide in which a 2'-OH group of a ribose has been modified, a nucleotide in which a phosphodiester bond between nucleosides has been modified, a nucleotide in which a ribose has been converted, a nucleotide in which a purine residue or a pyrimidine residue has been modified, a nucleotide containing an intramolecularly crosslinked ribose, and the like. Examples of the nucleotide in which a 2'-OH group of a ribose has been modified include a nucleotide containing 2'-O-methylribose, 2'-fluororibose, or the like. Examples of the nucleotide in which a phosphodiester bond between nucleosides has been modified include a nucleotide containing phosphorothioate, methylphosphonate, or the like, or a peptide-linked nucleic acid (PNA: peptide nucleic acid). The nucleotide in which a ribose has been converted includes a morpholino nucleic acid (PMO) and the like. The nucleotide in which a purine residue or a pyrimidine residue has been modified includes 5-methyl-deoxycytidine-phosphate (5-Me-dCMP), 2-amino-deoxyadenosine-phosphate (2-amino-dAMP), and a nucleotide containing C5-modified pyrimidine. The nucleotide containing an intramolecularly crosslinked ribose includes a locked nucleic acid (LNA) and the like. The polynucleotide of the present invention can be produced by a method known in the art based on the base sequence to be used and the type of modification.

A specific embodiment of the polynucleotide of the present invention is the same as the embodiment of the guide RNA of the present invention described in the above-mentioned sections <Guide RNA of Present Invention> and <Nucleic Acid Encoding Guide RNA of Present Invention>. Specific examples are shown below.

In some embodiments, the polynucleotide of the present invention is any of the following polynucleotides:
a polynucleotide which contains at least one functional region and an antisense region that is complementary to a portion of the target RNA and forms a double strand with the target RNA, wherein the at least one functional region is directly bound to the antisense region, and wherein the guide RNA does not substantially contain an ADAR-recruiting base sequence,
a polynucleotide which contains at least one functional region and an antisense region that is complementary to a portion of the target RNA and forms a double strand with the target RNA, wherein the at least one functional region is linked to the antisense region via a linker, and wherein the guide RNA does not substantially contain an ADAR-recruiting base sequence, and
a polynucleotide which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein the antisense region has a base to form a mismatched base pair with the target RNA.

In some embodiments, the polynucleotide of the present invention is a polynucleotide, which contains at least one functional region and an antisense region that is complementary to a portion of the target RNA and forms a double strand with the target RNA, wherein the at least one functional region is linked to the antisense region, wherein the guide RNA does not substantially contain an ADAR-recruiting base sequence, and wherein the polynucleotide has the following characteristic:
(1) the functional region is a region composed of a base sequence having any one or more functions among functions including stabilization of a guide RNA, localization of a guide RNA to the nucleus, localization of a guide RNA to the cytoplasm, promotion of double strand formation between the target RNA and the antisense region, inhibition of non-specific double strand formation by the antisense region, and stabilization of a complex formed of the target RNA and the antisense region,
(2) the functional region is a region composed of an snRNA sequence, a region composed of an rRNA sequence, a region composed of a base sequence to form a guanine quadruplex structure, or a region composed of a base sequence to form a stem-loop structure, or any combination thereof,
(3) the functional region is a region composed of an snRNA sequence and an optionally contained region composed of a base sequence to form a stem-loop structure,
(4) the functional region is a region composed of an snRNA sequence selected from a region composed of a U6 snRNA sequence, a region composed of a U1 snRNA sequence, and a region composed of a U7 snRNA sequence, and an optionally contained region composed of a base sequence to form a stem-loop structure,
(5) the functional region is a region composed of an rRNA sequence and an optionally contained region composed of a base sequence to form a stem-loop structure,
(6) the functional region is a region composed of a 5S rRNA sequence and an optionally contained region composed of a base sequence to form a stem-loop structure,
(7) the functional region is a region composed of a base sequence to form a guanine quadruplex structure or a region composed of a base sequence to form a stem-loop structure, or a combination thereof,
(8) the guide RNA is composed of an antisense region, a functional region composed of a base sequence to form a guanine quadruplex structure, an optionally contained additional functional region, and an optionally contained linker, and the antisense region, the functional region composed of a base sequence to form a guanine quadruplex structure, and the optionally contained additional functional region are linked in this order from the 5' side to the 3' side, or
(9) the guide RNA is composed of a functional region composed of a base sequence to form a stem-loop structure, an antisense region, an optionally contained additional functional region, and an optionally contained linker, and the functional region composed of a base sequence to form a stem-loop structure and the antisense region are linked in this order from the 5' side to the 3' side.

### <Pharmaceutical Composition of Present Invention>

The present invention also provides a pharmaceutical composition containing the guide RNA of the present invention, a nucleic acid encoding the guide RNA of the present invention (also referred to as "the nucleic acid of the present invention" in this section), the expression vector of the present invention, or the polynucleotide of the present invention, and a pharmaceutically acceptable excipient (also referred to as "the pharmaceutical composition of the present invention"). In some embodiments, the pharmaceutical composition of the present invention is a pharmaceutical composition containing the guide RNA of the present invention and a pharmaceutically acceptable excipient. In some embodiments, the pharmaceutical composition of the present invention is a pharmaceutical composition containing the guide RNA of the present invention, ADAR, and a pharmaceutically acceptable excipient. In some embodiments, the pharmaceutical composition of the present invention is a pharmaceutical composition containing the nucleic acid of the present invention and a pharmaceutically acceptable excipient. In some embodiments, the pharmaceutical composition of the present invention is a pharmaceutical composition containing the nucleic acid of the present invention, ADAR, and a pharmaceutically acceptable excipient. In some embodiments, the pharmaceutical composition of the present invention is a pharmaceutical composition that contains an expression vector containing a nucleic acid encoding the guide RNA of the present invention, and contains a pharmaceutically acceptable excipient. In some embodiments, the pharmaceutical composition of the present invention is a pharmaceutical composition that contains an expression vector containing a nucleic acid encoding the guide RNA of the present invention and containing a nucleic acid encoding ADAR, and contains a pharmaceutically acceptable excipient. In some embodiments, the pharmaceutical composition of the present invention is a pharmaceutical composition that contains an expression vector containing a nucleic acid encoding the guide RNA of the present invention, an expression vector containing a nucleic acid encoding ADAR, and a pharmaceutically acceptable excipient. In some embodiments, the pharmaceutical composition of the present invention is a pharmaceutical composition containing the polynucleotide of the present invention and a pharmaceutically acceptable excipient. In some embodiments, the pharmaceutical composition of the present invention is a pharmaceutical composition containing the polynucleotide of the present invention, ADAR, and a pharmaceutically acceptable excipient.

In some embodiments, the pharmaceutical composition of the present invention is a pharmaceutical composition that contains an expression vector containing a nucleic acid encoding the guide RNA of the present invention and containing a nucleic acid encoding human ADAR1 or human ADAR2, and contains a pharmaceutically acceptable excipient. In some embodiments, the pharmaceutical composition of the present invention is a pharmaceutical composition that contains an expression vector containing a nucleic acid encoding the guide RNA of the present invention, an expression vector containing a nucleic acid encoding human ADAR1 or human ADAR2, and a pharmaceutically acceptable excipient. In some embodiments, the pharmaceutical composition of the present invention is a pharmaceutical composition that contains an expression vector containing a nucleic acid encoding the guide RNA of the present invention and containing a nucleic acid composed of a base sequence encoding a polypeptide having the amino acid sequence represented by SEQ ID NO: 9 or a base sequence encoding a polypeptide composed of an amino acid sequence having 90% or more identity to the amino acid sequence and having adenosine deaminase activity, and contains a pharmaceutically acceptable excipient. In some embodiments, the pharmaceutical composition of the present invention is a pharmaceutical composition that contains an expression vector containing a nucleic acid encoding the guide RNA of the present invention, an expression vector containing a nucleic acid composed of a base sequence encoding a polypeptide having the amino acid sequence represented by SEQ ID NO: 9 or a base sequence encoding a polypeptide composed of an amino acid sequence having 90% or more identity to the amino acid sequence and having adenosine deaminase activity, and contains a pharmaceutically acceptable excipient.

The pharmaceutical composition of the present invention can be prepared by a commonly used method using an excipient usually used in the art, that is, a pharmaceutical excipient, a pharmaceutical carrier, or the like. Examples of the dosage form of such a pharmaceutical composition include parenteral preparations such as an injection and an agent for infusion, which can be administered by intravenous administration, subcutaneous administration, intradermal administration, intramuscular administration, or the like. In the formulation, an excipient, a carrier, an additive, or the like according to the dosage form can be used within a pharmaceutically acceptable range.

The dose of the guide RNA of the present invention, the nucleic acid of the present invention, the expression vector of the present invention, the polynucleotide of the present invention, the expression vector containing a nucleic acid encoding ADAR, or ADAR can be appropriately adjusted according to a cell in which the target RNA is present, the severity of symptoms or age of a patient, the dosage form of the formulation to be used, or the like. For example, the dose in the range of 0.001 mg/kg to 100 mg/kg can be adopted.

A disease that can be prevented or treated with the pharmaceutical composition of the present invention is, in some embodiments, a hereditary disease, in some embodiments, any disease in which editing of one or more adenosine or cytidine nucleosides in the target RNA brings about a beneficial change, and in some embodiments, any disease in which conversion of one or more adenosine nucleosides in the target RNA to inosine brings about a beneficial change. The pharmaceutical composition of the present invention can be used as a preventive or therapeutic agent for any disease in which editing of one or more adenosine or cytidine nucleosides in the target RNA brings about a beneficial change. In some embodiments, the pharmaceutical composition of the present invention can be used as a preventive or therapeutic agent for any disease in which conversion of one or more adenosine nucleosides in the target RNA to inosine brings about a beneficial change.

The present invention includes a pharmaceutical composition for preventing or treating any disease in which editing of one or more adenosine or cytidine nucleosides in the target RNA brings about a beneficial change, containing the guide RNA of the present invention, the nucleic acid of the present invention, the expression vector of the present invention, or the polynucleotide of the present invention. Further, the present invention includes a method for preventing or treating any disease in which editing of one or more adenosine or cytidine nucleosides in the target RNA brings about a beneficial change, including a step of administering a preventively effective amount or a therapeutically effective amount of the guide RNA of the present invention, the nucleic acid of the present invention, the expression vector of the present invention, or the polynucleotide of the present invention. Further, the present invention includes the guide RNA of the present invention, the nucleic acid of the present invention, the expression vector of the present invention, or the polynucleotide of the present invention for use in the prevention or treatment of any disease in which editing of one or more adenosine or cytidine nucleosides in the target RNA brings about a beneficial change. Further, the present invention includes use of the guide RNA of the present invention, the nucleic acid of the present invention, the expression vector of the present invention, or the polynucleotide of the present invention in the production of a pharmaceutical composition for preventing or treating any disease in which editing of one or more adenosine or cytidine nucleosides in the target RNA brings about a beneficial change.

### <Method for Editing Target RNA of Present Invention>

The present invention also provides a method for editing a target RNA using the guide RNA of the present invention (also referred to as "the editing method of the present invention"). In some embodiments, the editing method of the present invention includes a method in which the antisense region of the guide RNA of the present invention forms a double strand with the target RNA, ADAR is recruited to the formed double-stranded RNA, and the recruited ADAR converts adenosine of the target RNA sequence to inosine. Here, adenosine on the target RNA to be converted to inosine may form a mismatched base pair with the antisense region. In some embodiments, the editing method of the present invention includes a method in which the antisense region of the guide RNA of the present invention forms a double strand with the target RNA, ADAR is recruited to the formed double-stranded RNA, and the recruited ADAR converts cytidine of the target RNA sequence to uridine. Here, cytidine on the target RNA to be converted to uridine may form a mismatched base pair with the antisense region.

The editing method of the present invention includes (i) a step of introducing the guide RNA of the present invention, the nucleic acid encoding the guide RNA of the present invention, the expression vector of the present invention, or the polynucleotide of the present invention into a cell, a virus, or the like having a target RNA. In some embodiments, the editing method of the present invention further includes (ii) a step of introducing ADAR, a nucleic acid encoding ADAR, or an expression vector containing a nucleic acid encoding ADAR into the cell. The steps (i) and (ii) may be performed simultaneously or separately. In some embodiments, the introduction target cell is a eukaryotic cell, a prokaryote, or the like, and in some embodiments, the cell is a mammalian cell, a bacterium, or the like. In some embodiments, the introduction target virus includes a phage. In some embodiments, adenosine of the target RNA is converted to inosine, and in other embodiments, cytidine of the target RNA is converted to uridine. In addition, if the target RNA is in a coding region, the method may include a step of reading inosine as guanosine during translation.

### EXAMPLES

Unless otherwise specified, the steps described in the following Examples can be carried out according to a known method. In addition, for parts using a commercially available kit or reagent, or the like, an experiment was performed according to the attached protocol unless otherwise specified.

### Example 1: Production of Expression Plasmid for Guide RNA with U6 snRNA Sequence Added as Functional Region

A vector in which an H1 RNA polymerase III promoter (hereinafter referred to as H1 promoter) of a pSUPER.neo vector (Oligoengine, Inc., Catalog No. VEC-PBS-0004) was substituted with a human U6 snRNA polymerase III (hU6) promoter sequence (SEQ ID NO: 17) was constructed (hereinafter referred to as pSUPER.neo-U6 vector). A fragment containing the hU6 promoter sequence was amplified by a standard PCR method so as to add an EcoRI restriction enzyme site to the 5' side and a BglII restriction enzyme site to the 3' side using a pBAsi-hU6 Neo DNA having a hU6 promoter (Takara Bio Inc., Catalog No. 3227) as a template. In the reaction, a DNA polymerase Gflex (Takara Bio Inc., Catalog No. R060A) was used. The obtained hU6 promoter fragment was inserted into the pSUPER.neo vector using the restriction enzyme EcoRI site (5' side) and BglII site (3' side). E. coli DH5α Competent Cells (Takara Bio Inc., Catalog No. 9057, hereinafter referred to as "DH5α E. coli strain") were transformed with the constructed pSUPER.neo-U6 vector and subjected to liquid culture. The culture solution was centrifuged to recover the bacterial cells, and plasmid extraction and purification were performed using NucleoBond (registered trademark) Xtra Midi Plus EF (Takara Bio Inc., Catalog No. U0422B), and the amplified pSUPER.neo-U6 vector was obtained.

An expression plasmid for a guide RNA with a U6 snRNA sequence (hereinafter sometimes referred to as "U6") added as a functional region was constructed as follows. A DNA sequence (SEQ ID NO: 18) encoding a guide RNA U6-ASR-STL was inserted downstream of the hU6 promoter of the pSUPER.neo-U6 vector using the restriction enzyme BglII site (5' side) and HindIII site (3' side).

In the production of the DNA sequence to be inserted, a method of annealing a forward oligo and a reverse oligo (below) synthesized so as to form a restriction enzyme cleavage end was used.

Forward oligo: 5'-GATCT-DNA sequence encoding guide RNA-3'

Reverse oligo: 5'-AGCTT-reverse complementary sequence of DNA sequence encoding guide RNA-3'

The inserted DNA sequence contains a purine base (G or A) preferable for the transcription start site of polIII at the 5' side and a terminator sequence of polIII at the 3' side.

The produced guide RNA expression plasmid is referred to as pSUPERneo_U6_gRNA.

Here, ASR represents an antisense base sequence composed of 24 bases complementary to a portion of a detection reporter mRNA (Rluc-W104X) used in Example 4, and is composed of an RNA sequence encoded by a sequence from position 37 to position 60 of the DNA sequence represented by SEQ ID NO: 18. U6 is composed of the RNA sequence represented by SEQ ID NO: 1, and a nucleic acid encoding U6 is composed of the DNA sequence represented by SEQ ID NO: 10. STL is composed of the RNA sequence represented by SEQ ID NO: 2, and a nucleic acid encoding STL is composed of the DNA sequence represented by SEQ ID NO: 11.

The summary of the guide RNA is shown in Table 1. In Table 1, "5'-" indicates the functional region added to the 5' side of ASR, and "-3'" indicates the functional region added to the 3' side of ASR. "Linker" indicates a sequence that links ASR to each functional region.

The constructed pSUPERneo_U6_gRNA plasmid was multiplied in the same manner as described above using a DH5α E. coli strain (Takara Bio Inc., Catalog No. 9057).

### Example 2: Production of Expression Plasmid for Guide RNA with U1 snRNA Sequence, U7 snRNA Sequence, or 5S rRNA Sequence Added as Functional Region

An expression plasmid for a guide RNA with a U1 snRNA sequence, a U7 snRNA sequence, or a 5S rRNA sequence (hereinafter sometimes referred to as "U1", "U7", and "5S", respectively) added as a functional region was produced as follows. A corresponding promoter sequence and a DNA sequence encoding the guide RNA were inserted upstream of the H1 promoter of the pSUPER.neo vector using the restriction enzyme EcoRI site (5' side) and HindIII site (3' side). A DNA sequence encoding each of the following guide RNAs is linked immediately downstream of a human U1 snRNA promoter sequence (SEQ ID NO: 19), a human U7 snRNA promoter sequence (SEQ ID NO: 20), or a 5S rRNA promoter sequence (SEQ ID NO: 21).

### <Name of Guide RNA and SEQ ID NO of DNA Encoding the Same>

ASR-U1: SEQ ID NO: 22
ASR-U7: SEQ ID NO: 23
5S-ASR-STL: SEQ ID NO: 24
ASR-U1 and 5S-ASR-STL contain a poly-T sequence at the 3' side.

U1 is composed of the RNA sequence represented by SEQ ID NO: 3, and a nucleic acid encoding U1 is composed of the DNA sequence represented by SEQ ID NO: 12. ASR-U1 (SEQ ID NO: 22) is composed of a base sequence in which a sequence from position 3 to position 10 of SEQ ID NO: 12 has been substituted with ASR.

U7 is composed of the RNA sequence represented by SEQ ID NO: 4, and a nucleic acid encoding U7 is composed of the DNA sequence represented by SEQ ID NO: 13.

5S is composed of the RNA sequence represented by SEQ ID NO: 5, and a nucleic acid encoding 5S is composed of the DNA sequence represented by SEQ ID NO: 14.

The summary of the DNA sequence encoding each guide RNA is shown in Table 1.

When the DNA sequence to be inserted was produced, the production was performed by DNA synthesis (FASMAC). The obtained plasmids are referred to as pSUPERneo_U1_gRNA, pSUPERneo_U7_gRNA, and pSUPERneo_5S_gRNA, respectively.

The constructed plasmid was multiplied by the same method as in Example 1 using a DH5α E. coli strain (Takara Bio Inc., Catalog No. 9057).

### Example 3: Production of Expression Plasmid for Guide RNA with Gq Sequence and/or BoxB Sequence-Derived Sequence Added as Functional Region

An expression plasmid for a guide RNA with a Gq sequence (in Examples, the sequence to form 3Gq is sometimes referred to as a Gq sequence) and/or a BoxB sequence-derived sequence added as a functional region was produced as follows. A DNA sequence encoding each of 8 types of guide RNAs (see <Name of Guide RNA and SEQ ID NO of DNA Encoding the Same> described below) was inserted downstream of the H1 promoter of the pSUPER.neo vector using the restriction enzyme BglII site (5' side) and HindIII site (3' side).

### <Name of Guide RNA and SEQ ID NO of DNA Encoding the Same>

ASR-Gq: SEQ ID NO: 25
Gq-ASR: SEQ ID NO: 26
ASR-BoxB: SEQ ID NO: 27
BoxB-ASR: SEQ ID NO: 28
Gq-ASR-BoxB: SEQ ID NO: 29
BoxB-ASR-Gq: SEQ ID NO: 30
ASR-Gq-BoxB: SEQ ID NO: 31
Gq-BoxB-ASR: SEQ ID NO: 32

The inserted DNA sequence contains a purine base (G or A) preferable for the transcription start site of polIII at the 5' side and a terminator sequence of polIII at the 3' side.

Gq is composed of the RNA sequence represented by SEQ ID NO: 6, and a nucleic acid encoding Gq is composed of the DNA sequence represented by SEQ ID NO: 15. BoxB is composed of the RNA sequence represented by SEQ ID NO: 7, and a nucleic acid encoding BoxB is composed of the DNA sequence represented by SEQ ID NO: 16. The summary of each guide RNA is shown in Table 1.

The DNA sequence to be inserted was produced using the same method as in Example 1. The obtained guide RNA expression plasmids are collectively referred to as pSUPERneo_H1_gRNA.

The constructed plasmid was multiplied by the same method as in Example 1 using a DH5α E. coli strain (Takara Bio Inc., Catalog No. 9057).

A guide RNA to serve as a comparative control was produced in the same manner as described above using a DNA sequence (SEQ ID NO: 33) which does not contain a functional region and encodes only ASR. The inserted DNA sequence contains a purine base (G or A) preferable for the transcription start site of polIII at the 5' side and a terminator sequence of polIII at the 3' side.

**[Table 1]**

| | Functional region | | Name of guide RNA | 5'- | Linker | ASR | Linker | -3' |
|---|---|---|---|---|---|---|---|---|
| Comparative control | non | | ASR | - | - | ASR(24) | - | - |
| Example 1 | snRNA | U6 | U6-ASR-STL | U6 | GUCGAC | | UCUAGA | STL |
| Example 2 | | U1 | ASR-U1 | AU | - | | - | U1 |
| Example 2 | | U7 | ASR-U7 | - | - | | - | U7 |
| Example 2 | rRNA | 5S | 5S-ASR-STL | 5S | GUCGAC | | UCUAGA | STL |
| Example 3 | Gq | | ASR-Gq | - | - | | - | Gq |
| Example 3 | | | Gq-ASR | Gq | - | | - | - |
| Example 3 | BoxB | | ASR-BoxB | - | - | | UCU | BoxB |
| Example 3 | | | BoxB-ASR | BoxB | UCU | | - | - |
| Example 3 | Gq/BoxB | | Gq-ASR-BoxB | Gq | - | | UCU | BoxB |
| Example 3 | | | BoxB-ASR-Gq | BoxB | UCU | | - | Gq |
| Example 3 | | | ASR-Gq-BoxB | - | - | | - | Gq-UCU-BoxB |
| Example 3 | | | Gq-BoxB-ASR | Gq-BoxB | UCU | | - | - |

### Example 4: Production of Intracellular Target RNA Editing Activity Detection Reporter Expression Plasmid

As a reporter mRNA for detecting intracellular target RNA editing activity, a Renilla luciferase (Rluc) mRNA was used. The intracellular target RNA editing activity detection reporter Rluc mRNA (Rluc-W 104X) was designed based on an Rluc mRNA sequence encoded by an Rluc gene cloned into a psiCHECK-2 vector (Promega Corporation, Catalog No. C8021). Specifically, Rluc-W104X was designed such that G at position 311 of UGG encoding Trp which is an amino acid at position 104 of Rluc is substituted with A so as to encode a translation stop codon (UAG) in place of Trp. A Rluc-W104X expression plasmid was produced by substituting a psiCHECK-2 vector (Promega Corporation, Catalog No. C8021) with a DNA sequence (SEQ ID NO: 34) containing a mutation site G311A using the restriction enzyme DraIII site (5' side) and AatII site (3' side). The obtained plasmid is referred to as psiCHECK-2_Rluc-W104X. The constructed psiCHECK-2_Rluc-W104X vector was multiplied by the same method as in Example 1 using a DH5α E. coli strain (Takara Bio Inc., Catalog No. 9057).

### Example 5: Production of ADAR2 Expression Plasmid

An ADAR2 expression plasmid was produced by inserting a DNA sequence (SEQ ID NO: 8) encoding ADAR2 (SEQ ID NO: 9) at the restriction enzyme EcoRI site (5' side) and BamHI site (3' side) downstream of a CMV-derived promoter of a pAAV-CMV vector (Takara Bio Inc., Catalog No. 6230) using the restriction enzyme EcoRI site (5' side) and BglII site (3' side). The obtained plasmid is referred to as pAAV-CMV-ADAR2 In the inserted DNA sequence, a Kozak sequence ("ACC" from position 13 to position 15 of SEQ ID NO: 8) was placed upstream of the translation start codon, and a stop codon was placed downstream of the ADAR2 gene. The constructed pAAV-CMV-ADAR2 plasmid was multiplied by the same method as in Example 1 using a DH5α E. coli strain (Takara Bio Inc., Catalog No. 9057).

### Example 6: Transfection

### Experiment 1 (Luciferase assay whose results are shown in Table 2 and transfection for examining RNA editing efficiency by Sanger sequencing)

Human embryonic kidney-derived cell line HEK 293 cells were suspended in Dulbecco's Modified Eagle's Medium (DMEM; Thermo Fisher Scientific, Inc., Catalog No. 10569-010) supplemented with 5% fetal bovine serum (FBS), and inoculated in a 96-well plate at 2.0 × 10⁴ cells/100 µL and cultured overnight at 37°C in the presence of 5% CO₂.

The intracellular target RNA editing activity detection reporter expression plasmid (psiCHECK-2_Rluc-W104X) produced in Example 4, the ADAR2 expression plasmid (pAAV-CMV-ADAR2) produced in Example 5, the guide RNA expression plasmid (pSUPERneo_U6_gRNA, pSUPERneo_U1_gRNA, pSUPERneo_U7_gRNA, pSUPERneo_5S_gRNA, or pSUPERneo_H1_gRNA) produced in Example 1, 2, or 3, and a carrier plasmid were mixed at a weight ratio of 1:40:30:9 so that the total amount was 100 ng/well. As the carrier plasmid, a pHelper vector (Takara Bio Inc., Catalog No. 6230) was used.

The HEK 293 cells cultured overnight after inoculation were transfected using Lipofectamine^{™} 3000 Transfection Reagent (Thermo Fisher Scientific, Inc., Catalog No. L3000015). The guide RNA expression plasmid to serve as a comparative control was also transfected in the same manner.

After transfection, the cells were cultured under the same conditions, and the cells after 3-day culture were subjected to an examination of RNA editing activity by a luciferase assay described in Example 7 and an RNA editing efficiency by Sanger sequencing described in Example 8.

### Experiment 2 (Luciferase assay whose results are shown in Table 3 and transfection for examining RNA editing efficiency by Sanger sequencing)

The intracellular target RNA editing activity detection reporter expression plasmid (psiCHECK-2_Rluc-W104X), the ADAR2 expression plasmid (pAAV-CMV-ADAR2), the guide RNA expression plasmid (pSUPERneo_U6_gRNA or pSUPERneo_H1_gRNA) produced in Example 1 or 3, and a carrier plasmid were mixed at a weight ratio of 1:20:15:4 so that the total amount was 100 ng/well, and transfection into HEK 293 cells was performed using the same method as in Experiment 1. As the carrier plasmid, a pHelper vector (Takara Bio Inc., Catalog No. 6230) was used.

### Example 7: Detection of RNA Editing Activity by Luciferase Assay

In each well of cells transfected in Example 6, a firefly luciferase luminescence intensity (Fluc) to correct the transfection efficiency and a Renilla luciferase luminescence intensity (Rluc) to evaluate the editing activity were measured using Dual-Glo (registered trademark) Luciferase Assay System (Promega Corporation, Catalog No. E2940) and EnVision (PerkinElmer, Inc.) according to the attached protocol.

Table 2 shows the results obtained by the transfection experiment of Experiment 1 of Example 6.

In Table 2, a Rluc/Fluc value was calculated to correct the transfection efficiency among the samples, and the Rluc/Fluc value of each guide RNA sample is expressed as a relative value when the Rluc/Fluc value of ASR used as the comparative control was assumed to be 1. An arithmetic mean of 4 independent trials is shown.

**[Table 2]**

| Functional region | | Name of guide RNA | Relative value of Rluc/Fluc value | RNA editing efficiency (%) |
|---|---|---|---|---|
| non (Comparative control) | | ASR | 1 | N.D. |
| snRNA | U6 | U6-ASR-STL | 3.06 | 28.5 |
| | U1 | ASR-U1 | 2.04 | 23.0 |
| | U7 | ASR-U7 | 2.60 | 32.9 |
| rRNA | 5S | 5S-ASR-STL | 6.76 | 52.6 |
| Gq | | ASR-Gq | 2.97 | 29.5 |
| | | Gq-ASR | 0.91 | N.D. |
| BoxB | | ASR-BoxB | 0.54 | N.D. |
| | | BoxB-ASR | 4.42 | 33.8 |
| Gq/BoxB | | Gq-ASR-BoxB | 0.41 | N.D. |
| | | BoxB-ASR-Gq | 0.68 | N.D. |
| | | ASR-Gq-BoxB | 2.82 | 23.6 |
| | | Gq-BoxB-ASR | 0.58 | N.D. |

| | | | | |
|---|---|---|---|---|
| "N.D." represents the detection limit or less. | | | | |

Table 3 shows the results obtained by the transfection experiment of Experiment 2 of Example 6.

In Table 3, a Rluc/Fluc value was calculated to correct the transfection efficiency among the samples, and the arithmetic mean value and the standard deviation value of three wells, and the Rluc/Fluc value of each guide RNA sample expressed as a relative value when the Rluc/Fluc value of ASR used as the comparative control was assumed to be 1 are shown.

**[Table 3]**

| Functional region | | Name of guide RNA | Rluc/Fluc value | | | RNA editing efficiency (%) |
|---|---|---|---|---|---|---|
| | | | Relative value | Arithmetic mean value | Standard deviation value | |
| non (Comparative control) | | ASR | 1 | 0.026 | 0.0010 | 11.2 |
| snRNA | U6 | U6-ASR-STL | 6.96 | 0.181 | 0.0119 | 71.3 |
| Gq | | ASR-Gq | 6.23 | 0.162 | 0.0040 | 58.7 |
| | | Gq-ASR | 2.42 | 0.063 | 0.0017 | 34.2 |
| BoxB | | ASR-BoxB | 1.58 | 0.041 | 0.0023 | 20.2 |
| | | BoxB-ASR | 4.96 | 0.129 | 0.0102 | 47.2 |
| Gq/BoxB | | Gq-ASR-BoxB | 0.65 | 0.017 | 0.0011 | 12.9 |
| | | BoxB-ASR-Gq | 1.15 | 0.030 | 0.0029 | 17.0 |
| | | ASR-Gq-BoxB | 6.69 | 0.174 | 0.0039 | 68.4 |

When the editing of the target RNA from A to I is induced in a cell by the ADAR protein and the guide RNA, the mutated translation stop codon (UIG) becomes Trp when translated from the mRNA, and the luminescence of Renilla luciferase is restored. That is, it is shown that the higher the Rluc value is, the higher the RNA editing activity is.

From Table 2, the guide RNA in which an snRNA or 5S rRNA is linked to the antisense region, the guide RNA in which only Gq is linked to the antisense region at the 3' side, the guide RNA in which only BoxB is linked to the antisense region at the 5' side, and the guide RNA in which Gq and BoxB are linked to the antisense region at the 3' side showed a relative value of the Rluc/Fluc value of 2.0 or more with respect to the guide RNA (ASR) composed of the antisense region used as the comparative control.

From Table 3, all guide RNAs except the guide RNA in which Gq is linked to the antisense region at the 5' side and BoxB is linked thereto at the 3' side showed a higher Rluc/Fluc value than ASR used as the comparative control. In particular, the guide RNA in which U6 is linked to the antisense region, the guide RNA in which only Gq is linked to the antisense region at the 3' side, the guide RNA in which only BoxB is linked to the antisense region at the 5' side, and the guide RNA in which Gq and BoxB are linked to the antisense region at the 3' side showed a relative value of the Rluc/Fluc value of 5.0 or more with respect to ASR used as the comparative control.

### Example 8: Examination of RNA Editing Efficiency by Sanger Sequencing

The cells transfected in Example 6 were recovered, and the total RNA was extracted and purified from the recovered cells using QIAshredder (QIAGEN N.V, Catalog No. 79656), RNeasy Mini Kit (QIAGEN N.V, Catalog No. 74106), and RNase-free DNase Set (QIAGEN N.V, Catalog No. 79254) according to the attached protocol. The purified RNA was subjected to a DNA digestion treatment again using Recombinant DNase I (RNase-free) (Takara Bio Inc., Catalog No. 2270A) according to the attached protocol. The obtained RNA was subjected to a reverse transcription reaction using SuperScript (registered trademark) VILO^{™} cDNA Synthesis kit (Thermo Fisher Scientific, Inc., Catalog No. 11754-250) according to the attached protocol, thereby obtaining a cDNA. The cDNA was used as a template, and a PCR reaction was performed using the following primers for amplifying a fragment containing an editing site and PrimeSTAR (registered trademark) GXL DNA Polymerase (Takara Bio Inc., Catalog No. R050A) according to the attached protocol.

### <Primers Used in Experiment in Table 2>

Forward primer: ATGGCTTCCAAGGTGTACGACC (SEQ ID NO: 35)
Reverse primer: TTACTGCTCGTTCTTCAGCACG (SEQ ID NO: 36) <Primers Used in Experiment in Table 3>
Forward primer: CTCGCTGCAAGCAAATGAAC (SEQ ID NO: 37)
Reverse primer: TCGTCCCAGGACTCGATCAC (SEQ ID NO: 38)

The PCR-multiplied fragment was purified using ExoSAP-IT^{™} Express PCR Product Cleanup Reagents (Thermo Fisher Scientific, Inc., Catalog No. 75001.200.UL) according to the attached protocol, and subjected to a Sanger sequencing reaction by a 3730 xl DNA analyzer (Applied Biosystems, Inc.) together with the forward primer used in the PCR multiplication.

Table 2 shows the results obtained by the transfection experiment of Experiment 1 of Example 6.

A waveform data file (extension: .ab1) obtained by the Sanger sequencing reaction was analyzed using QSVanalyzer (Bioinformatics, 2009, Vol. 25, pp. 3244-3250), and the signal intensity ratio of guanine (G) (G / (G+A) × 100) was defined as the RNA editing efficiency (%). "N.D." represents the detection limit or less. The results of representative examples are shown.

Table 3 shows the results obtained by the transfection experiment of Experiment 2 of Example 6.

In Table 3, the obtained waveform data file (extension: .abl) was analyzed using QSVanalyzer. The corrected signal intensity ratio of G (G / (G+A) × 100) when the signal intensity of G of unedited Rluc-W104X was corrected to 0, the signal intensity of A thereof was corrected to 1, the signal intensity of G of wild-type Rluc was corrected to 1, and the signal intensity of A thereof was corrected to 0 was defined as the RNA editing efficiency (%). The results of representative examples are shown.

From Table 2, the RNA editing efficiency induced by ASR used as the comparative control was equal to or less than the detection limit. Under the same conditions, the guide RNA in which an snRNA or 5S rRNA is linked to the antisense region, the guide RNA in which only Gq is linked to the antisense region at the 3' side, the guide RNA in which only BoxB is linked to the antisense region at the 5' side, and the guide RNA in which Gq and BoxB are linked to the antisense region at the 3' side showed an RNA editing efficiency of 23% or more.

The guide RNA of the present invention containing the antisense region to which the functional region shown in Table 3 is linked showed a higher RNA editing efficiency than ASR used as the comparative control. In particular, the guide RNA in which U6 is linked to the antisense region, the guide RNA in which only Gq is linked to the antisense region at the 3' side, the guide RNA in which only BoxB is linked to the antisense region at the 5' side, and the guide RNA in which Gq and BoxB are linked to the antisense region at the 3' side showed an RNA editing efficiency of 47% or more.

### Example 9: Examination of RNA Editing Efficiency Targeting Firefly Luciferase (Fluc) mRNA

It was confirmed whether the guide RNA of the present invention shows a high RNA editing efficiency even for different target RNAs.

A guide RNA expression plasmid in which A at position 167 in the coding region of firefly luciferase (Fluc) cloned into a psiCHECK-2 vector (Promega Corporation, Catalog No. C8021) was determined to be the editing target was produced in the same manner as in Examples 1 and 3. The DNA sequences encoding the guide RNA used for the production are as follows.

### <Name of Guide RNA and SEQ ID NO of DNA Encoding the Same>

U6-ASRf-STL: SEQ ID NO: 39
ASRf-Gq: SEQ ID NO: 40
Gq-ASRf: SEQ ID NO: 41
ASRf-BoxB: SEQ ID NO: 42
BoxB-ASRf: SEQ ID NO: 43
Gq-ASRf-BoxB: SEQ ID NO: 44
BoxB-ASRf-Gq: SEQ ID NO: 45
ASRf-Gq-BoxB: SEQ ID NO: 46
Gq-BoxB-ASRf: SEQ ID NO: 47

The DNA sequence contains a purine base (G or A) preferable for the transcription start site of polIII at the 5' side and a terminator sequence of polIII at the 3' side.

The obtained plasmids are collectively referred to as pSLIPERneo_gRNAf.

ASRf represents an antisense base sequence composed of 24 bases complementary to the Fluc reporter mRNA containing the editing target A, and is composed of an RNA base sequence encoded by a sequence from position 3 to position 26 of the DNA sequence represented by SEQ ID NO: 40.

A guide RNA expression plasmid to serve as a comparative control was also produced in the same manner using a DNA sequence (SEQ ID NO: 48) that does not contain a functional region and encodes only ASRf.

In the same manner as in Experiment 1 of Example 6, a Fluc reporter expression plasmid (psiCHECK-2 (Promega Corporation, C8021), the ADAR2 expression plasmid (pAAV-CMV-ADAR2) produced in Example 5, the guide RNA expression plasmid (pSUPERneo_gRNAf), and a carrier plasmid were mixed at a weight ratio of 1:80:100:19 so that the total amount was 100 ng/well, and HEK 293 cells were transfected therewith using Lipofectamine^{™} 3000 Transfection Reagent (Thermo Fisher Scientific, Inc., Catalog No. L3000015). The guide RNA expression plasmid to serve as a comparative control was also transfected in the same manner. As the carrier plasmid, a pHelper vector (Takara Bio Inc., Catalog No. 6230) was used.

A PCR reaction was performed in the same manner as in Example 8 (in the case of Table 2), and a Sanger sequencing reaction was performed using sequence primers. The RNA editing efficiency targeting the Fluc reporter mRNA was calculated from the waveform data file of the Sanger sequencing reaction in the same manner as for Table 2. The primers used for the PCR reaction and the Sanger sequencing reaction of this test are shown below.
Forward Primer Fluc: ACCCGCTTAAAAGCTTGGC (SEQ ID NO: 49)
Reverse primer Fluc: CCACGGTAGGCTGAGAAATG (SEQ ID NO: 50)
Sequence primer: CATGCTGTTCAGCAGCTCGC (SEQ ID NO: 51)

An arithmetic mean of 3 independent trials is shown in Table 4.

**[Table 4]**

| Functional region | | Name of guide RNA | RNA editing efficiency (%) |
|---|---|---|---|
| non (Comparative control) | | ASRf | 5.7 |
| snRNA | U6 | U6-ASRf-STL | 17.5 |
| Gq | | ASRf-Gq | 19.5 |
| | | Gq-ASRf | 7.3 |
| BoxB | | A SRf-BoxB | 8.9 |
| | | BoxB-ASRf | 5.1 |
| Gq/BoxB | | Gq-ASRf-BoxB | 4.2 |
| | | BoxB-ASRf-Gq | 18.2 |
| | | ASRf-Gq-BoxB | 9.2 |
| | | Gq-BoxB-ASRf | 2.5 |

The guide RNA in which U6 is linked to the antisense region, the guide RNA in which only Gq is linked to the antisense region at the 5' side or the 3' side, the guide RNA in which only BoxB is linked to the antisense region at the 3' side, the guide RNA in which BoxB is linked to the antisense region at the 5' side and Gq is linked thereto at the 3' side, and the guide RNA in which Gq and BoxB are linked to the antisense region at the 3' side showed a higher RNA editing efficiency than ASRf used as the comparative control. In particular, the guide RNA in which U6 is linked to the antisense region, the guide RNA in which only Gq is linked to the antisense region at the 3' side, and the guide RNA in which BoxB is linked to the antisense region at the 5' side and Gq is linked thereto at the 3' side showed an RNA editing efficiency of 17% or more.

### Example 10: Examination of Intracellular Stability of Guide RNA

The intracellular stability of a guide RNA can be evaluated by comparing the residual amount of the guide RNA after transcription inhibition. It means that the larger the residual amount of a target guide RNA after the introduction of the guide RNA into a cell is, the higher the stability is.

HEK 293 cells were cultured in DMEM supplemented with 5% FBS, and inoculated in a 24-well plate at 1 × 10⁵ cells/0.5 mL and cultured overnight at 37°C in the presence of 5% CO₂. On the following day, the intracellular target RNA editing activity detection reporter expression plasmid (psiCHECK-2_Rluc-W104X) produced in Example 4, the ADAR2 expression plasmid (pAAV-CMV-ADAR2) produced in Example 5, the guide RNA expression plasmid (pSUPERneo_H1_gRNA or pSUPERneo_U6_gRNA) produced in Example 1 or 3, and a carrier plasmid were mixed at a weight ratio of 1:40:30:9 so that the total amount was 400 ng/well. As the carrier plasmid, a pHelper vector (Takara Bio Inc., Catalog No. 6230) was used. The HEK 293 cells were transfected using Lipofectamine^{™} 3000 Transfection Reagent (Thermo Fisher Scientific, Inc., Catalog No. L3000015).

24 hours after transfection, the cells in some wells were recovered, and the state before addition of actinomycin D was determined to be 0 hours. At the same time, in the wells from which cells were not recovered, the culture solution was replaced with DMEM supplemented with actinomycin D (Wako Pure Chemical Industries, Ltd., Catalog No. 010-21263) at 5 µg/mL. Actinomycin D is known to bind to a double-stranded DNA and inhibit transcription by RNA polymerase. The cells were recovered at 4 hours and 6 hours after the addition of actinomycin D, and the total RNA containing a guide RNA was extracted and purified from the recovered cells using miRNeasy Mini Kit (QIAGEN N.V, Catalog No. 217004) and RNase-free DNase Set (QIAGEN N.V, Catalog No. 79254) according to the attached protocol. The obtained RNA was subjected to polyA addition to a small RNA using Mir-X miRNAFirst-Strand Synthesis Kit (Takara Bio Inc., Catalog No. 638315) according to the attached protocol, and then a reverse transcription reaction was performed, thereby obtaining a cDNA. The cDNA was used as a template, and two types of touchdown PCR reactions were performed using PrimeSTAR (registered trademark) GXL DNA Polymerase (Takara Bio Inc., Catalog No. R050A). In the PCR reaction of U6 snRNA that is endogenously expressed in cells, U6 Forward Primer and U6 Reverse Primer attached to Mir-X miRNA qRT-PCR TB Green (registered trademark) Kit (Takara Bio Inc., Catalog No. 638314) were used. The amount of intracellular U6 snRNA was used to correct the amount of the guide RNA among samples. In the PCR reaction of the guide RNA, a primer having a sequence (SEQ ID NO: 52) homologous to ASR and mRQ 3' Primer attached to the above kit were used. The touchdown PCR reaction conditions are composed of a pre-cycle thermal denaturation stage at 94°C for 1 minute, an amplification stage including thermal denaturation at 98°C for 10 seconds, annealing at 65°C for 15 seconds, and an extension reaction at 68°C for 30 seconds, and an extension reaction stage at 72°C for 3 minutes. Among these, at the first to fifth cycles in the amplification stage, a touchdown method in which the annealing temperature is lowered by 1°C after each passage of the cycle is employed, and the annealing temperature at the sixth cycle and thereafter is maintained at 60°C. The PCR multiplication stage of the intracellular U6 snRNA was performed for a total of 23 cycles, and the PCR multiplication stage of the guide RNA was performed for a total of 20 cycles.

Each PCR product was electrophoresed using a 2% agarose gel, and the electrophoresis results were photographed using ChemiDoc^{™} Touch Imaging System (Bio-Rad Laboratories, Inc.). The PCR bands were quantified using Image Lab 6.1 Software for Windows (Bio-Rad Laboratories, Inc.).

In Table 5, a value obtained by correcting the signal intensity of the PCR band using the guide RNA as the template by the signal intensity of the PCR band using the intracellular U6 snRNA as the template (guide RNA/U6 value) was calculated. As the residual amount of the guide RNA, the value before the addition of actinomycin D was assumed to be 1, and the relative value of the guide RNA/U6 value at 4 hours and 6 hours after the addition of actinomycin D is shown. An arithmetic mean of 3 independent trials is shown.

**[Table 5]**

| Functional region | | Name of guide RNA | Residual amount of guide RNA | |
|---|---|---|---|---|
| | | | 4 hours | 6 hours |
| non (Comparative control) | | ASR | 0.36 | 0.40 |
| snRNA | U6 | U6-ASR-STL | 0.46 | 0.52 |
| Gq | | ASR-Gq | 0.48 | 0.59 |
| BoxB | | BoxB-ASR | 0.57 | 0.68 |

It means that the larger the residual amount of a guide RNA after the addition of actinomycin D is, the higher the intracellular stability of the guide RNA is.

The guide RNA of the present invention shown in Table 5 showed a high residual amount of the guide RNA at 4 hours and 6 hours after the addition of actinomycin D as compared with ASR used as the comparative control, and was confirmed to have high stability.

### INDUSTRIAL APPLICABILITY

The guide RNA of the present invention is useful for editing a target RNA, and a pharmaceutical composition containing the guide RNA of the present invention, a nucleic acid encoding the guide RNA of the present invention, the expression vector of the present invention, or the polypeptide of the present invention is useful in that it can be used for various diseases that can be prevented or treated by editing a target RNA, including hereditary diseases.

### SEQUENCE LISTING FREE TEXT

SEQ ID NOS: 1 to 7: Synthetic RNA
SEQ ID NOS: 10 to 52: Synthetic DNA

## Claims

1. A guide RNA for editing a target RNA by ADAR, comprising at least one functional region and an antisense region that is complementary to a portion of the target RNA and can form a double strand with the target RNA, wherein the at least one functional region is linked to the antisense region, and wherein the guide RNA does not substantially contain an ADAR-recruiting base sequence.

2. The guide RNA according to claim 1, wherein the at least one functional region is directly bound to the antisense region or linked to the antisense region via a linker.

3. The guide RNA according to claim 1 or 2, wherein the antisense region comprises a base to form a mismatched base pair with the target RNA.

4. The guide RNA according to any one of claims 1 to 3, wherein the functional region is a region including a base sequence having any one or more functions among functions including stabilization of the guide RNA, localization of the guide RNA to a nucleus, localization of the guide RNA to cytoplasm, promotion of double strand formation between the target RNA and the antisense region, inhibition of non-specific double strand formation by the antisense region, and stabilization of a complex formed of the target RNA and the antisense region.

5. The guide RNA according to any one of claims 1 to 4, wherein the functional region is a region composed of an snRNA sequence, a region composed of an rRNA sequence, a region composed of a base sequence to form a guanine quadruplex structure, or a region composed of a base sequence to form a stem-loop structure, or any combination thereof.

6. The guide RNA according to claim 5, wherein the functional region is a region composed of an snRNA sequence and an optionally contained region composed of a base sequence to form a stem-loop structure.

7. The guide RNA according to claim 6, wherein the region composed of an snRNA sequence is a region composed of a U6 snRNA sequence, a region composed of a U1 snRNA sequence, or a region composed of a U7 snRNA sequence.

8. The guide RNA according to claim 5, wherein the functional region is a region composed of an rRNA sequence and an optionally contained region composed of a base sequence to form a stem-loop structure.

9. The guide RNA according to claim 8, wherein the region composed of an rRNA sequence is a region composed of a 5S rRNA sequence.

10. The guide RNA according to claim 5, wherein the functional region is a region composed of a base sequence to form a guanine quadruplex structure, or a region composed of a base sequence to form a stem-loop structure, or a combination thereof.

11. The guide RNA according to any one of claims 1 to 5,
wherein the guide RNA comprises the antisense region, the functional region composed of a base sequence to form a guanine quadruplex structure, an optionally contained additional functional region, and an optionally contained linker, and
wherein the antisense region, the functional region composed of a base sequence to from a guanine quadruplex structure, and the optionally contained additional functional region are linked in this order from the 5' side to the 3' side.

12. The guide RNA according to any one of claims 1 to 5,
wherein the guide RNA comprises a functional region composed of a base sequence to form a stem-loop structure, the antisense region, an optionally contained additional functional region, and an optionally contained linker, and
wherein the functional region composed of a base sequence to form a stem-loop structure and the antisense region are linked in this order from the 5' side to the 3' side.

13. A system for editing a target RNA, comprising the guide RNA according to any one of claims 1 to 12 and ADAR

14. A nucleic acid, encoding the guide RNA according to any one of claims 1 to 12.

15. An expression vector, comprising a nucleic acid encoding the guide RNA according to any one of claims 1 to 12.

16. The expression vector according to claim 15, further comprising a nucleic acid encoding ADAR.

17. The expression vector according to claim 16, wherein the ADAR is ADAR1 or ADAR2

18. A host cell into which the expression vector according to any one of claims 15 to 17 has been introduced.

19. A method for producing an expression vector, comprising culturing the host cell according to claim 18.

20. A pharmaceutical composition, comprising the expression vector according to any one of claims 15 to 17 and a pharmaceutically acceptable excipient.

21. A pharmaceutical composition, comprising the expression vector according to claim 15, an expression vector containing a nucleic acid encoding ADAR, and a pharmaceutically acceptable excipient.

22. A method for editing a target RNA, comprising introducing a nucleic acid encoding the guide RNA according to any one of claims 1 to 12 into a cell, a virus, or the like.

23. A polynucleotide for editing a target RNA by ADAR, comprising at least one functional region and an antisense region that is complementary to a portion of the target RNA and can form a double strand with the target RNA, wherein the at least one functional region is linked to the antisense region, and wherein the guide RNA does not substantially contain an ADAR-recruiting base sequence.
